(19) Europäisches Patentamt
European Patent Office
Office européen des brevets

(11) Veröffentlichungsnummer: **0 180 539**
**A1**

# (12) EUROPÄISCHE PATENTANMELDUNG

(21) Anmeldenummer: **85810414.4**

(22) Anmeldetag: **12.09.85**

(51) Int. Cl.⁴: **C 07 D 493/22**, A 01 N 43/22, C 07 F 7/18 // (C07D493/22, 313:00, 311:00, 311:00, 307:00)

(30) Priorität: **18.09.84 CH 4457/84**

(43) Veröffentlichungstag der Anmeldung: **07.05.86 Patentblatt 86/19**

(84) Benannte Vertragsstaaten: **AT BE CH DE FR GB IT LI LU NL SE**

(71) Anmelder: **CIBA-GEIGY AG, Klybeckstrasse 141, CH-4002 Basel (CH)**

(72) Erfinder: **Frei, Bruno, Dr., Breitibachstrasse 10, CH-8600 Dübendorf (CH)**
Erfinder: **O'Sullivan, Anthony Cornelius, Dr., Habsburgerstrasse 38, CH-4055 Basel (CH)**
Erfinder: **Maienfisch, Peter, Dr., Traugott Meyer-Strasse 5, CH-4147 Aesch (CH)**

(54) **13-Halo- und 13-Hydroximilbemycine.**

(57) Verbindungen der Formel I

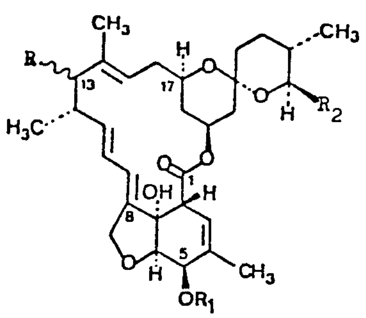

die mit R in 13-Position α- oder β-ständig halogeniert oder hydroxiliert sind, und worin $R_1$ Wasserstoff, eine Silyl- oder Acylgruppe bedeutet und $R_2$ Methyl, Ethyl, Isopropyl oder sek.-Butyl ist, lassen sich nach Verfahren gewinnen, die von entsprechend substituierten Δ13,14-15-hydroxi-Milbemycin-Derivaten der Formel II oder ihren Folgeprodukten ausgehen. Verbindungen der Formel I lassen sich zur Bekämpfung von Endo- und Ektoparasiten einsetzen, vor allem gegen tierparasitäre Nematoden.

EP 0 180 539 A1

0180539



CIBA-GEIGY AG 5-15074/+

Basel (Schweiz)

13-Halo- und 13-Hydroximilbemycine

Die vorliegende Erfindung betrifft Verfahren zur Herstellung von Milbemycin-Derivaten der Formel I und die Verwendung dieser Produkte zur Bekämpfung von Schädlingen wie Ekto- und Endoparasiten .

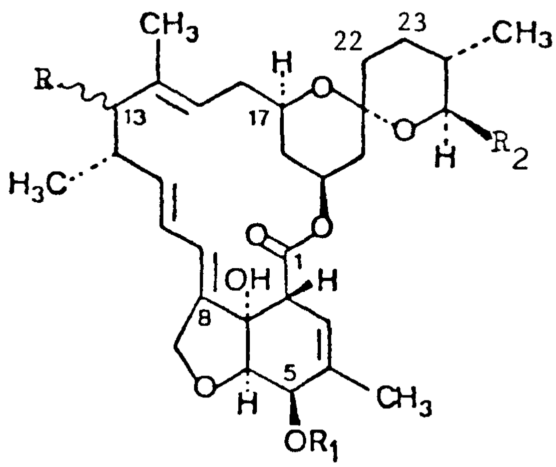

I

In der Formel I bedeuten

$R_1$ Wasserstoff, eine Silyl- oder Acylgruppe,

$R_2$ Methyl, Ethyl, Isopropyl oder sek.Butyl, und

R Fluor, Chlor, Brom, Jod oder eine Hydroxigruppe.

Verbindungen, worin $R_2$ sek.Butyl darstellt, sollen hier und im folgenden gleichfalls zu den Milbemycin-Derivaten gerechnet werden, obwohl sie nach der üblichen Systematik nicht darunter fallen, sondern gemäss US-PS 4.173.571 von Avermectin-Derivaten abgeleitet sind.

Im Rahmen vorliegender Erfindung sind Verbindungen der Formel I bevorzugt, worin $R_1$ Wasserstoff bedeutet. Acyl- und Silylgruppen des Substituenten $R_1$ sind im wesentlichen als Schutzgruppen aufzufassen, deren Anwesenheit aber den biologischen Wert der zugrundeliegenden Verbindungen mit $R_1$ = H nicht mindert.

Als Acylgruppe für $R_1$ kommen $R_3$-CO-Reste und $R_4$-$SO_2$-Reste in Frage, wobei $R_3$ vorzugsweise einen unsubstituierten oder halogenierten $C_1$-$C_6$-aliphatischen Rest oder unsubstituiertes oder durch $C_1$-$C_4$-Alkyl oder Halogen substituiertes Phenyl bedeutet und $R_4$ einen $C_1$-$C_4$-Alkylrest oder einen unsubstituierten oder durch Methyl, Chlor oder Nitro substituierten Phenylrest darstellt.

Bevorzugt sind Verbindungen der Formel I, worin $R_1$ Wasserstoff, einen $R_3$-CO-Rest oder $R_4$-$SO_2$-Rest bedeutet, in welchen $R_3$ einen $C_1$-$C_4$-Alkylrest oder eine unsubstituierte oder durch Methyl oder Chlor substituierte Phenylgruppe darstellt und $R_4$ Methyl, Ethyl, Phenyl, p-Tolyl, o-Nitrophenyl, p-Chlorphenyl ist, während $R_2$ Methyl, Ethyl, Isopropyl oder sek.Butyl bedeutet.

Als Beispiele für $R_3$, die keine Limitierungen darstellen sollen, seien genannt Methyl, Ethyl, Propyl, Isopropyl, tert.Butyl, Phenyl, p-Chlorphenyl, p-Tolyl.

Als Silyl-Gruppen kommen solche der Formel

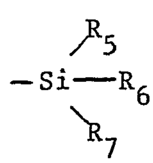

in Frage, worin $R_5$ einen $C_1$-$C_4$-aliphatischen Rest oder Benzyl und $R_6$ und $R_7$ unabhängig voneinander einen $C_1$-$C_4$-aliphatischen Rest, Benzyl oder Phenyl bedeuten.

Eine andere Gruppe bevorzugter Verbindungen der Formel I sind solche, worin $R_1$ Wasserstoff oder die vorgenannte Silylgruppe darstellt, in der $R_5$ Methyl, Ethyl, Propyl, Isopropyl, tert.Butyl, und $R_6$ und $R_7$ unabhängig Methyl, Ethyl, Isopropyl, tert.Butyl, Phenyl oder Benzyl bedeuten, während $R_2$ Methyl, Ethyl, Isopropyl oder sek.Butyl ist.

Beispiele für Silyl-Gruppen sind Trimethylsilyl, tris(tert.-Butyl) silyl, Diphenyl-tert.butylsilyl, bis(Isopropyl)methylsilyl und besonders tert.Butyl-dimethylsilyl.

Der Substituent R in 13-Position bedeutet in natürlich vorkommenden Milbemycinen ($R_1$ = H; $R_2$ = $CH_3$, $C_2H_5$ oder iso$C_3H_7$) stets Wasserstoff. Bei Avermectinen dagegen steht in der 13-Position ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen.

Durch Hydrolyse des Zucker-Restes der Avermectine gelangt man leicht zu den entsprechenden Avermectin-aglykonen, die eine allylische 13α-Hydroxy-Gruppe besitzen. In "Tetrahedron Letters", Vol. 24, No. 48, pp. 5333-5336 [1983] wird beschrieben, wie sich aus geschütztem (Silylierung in 5-Position) 22,23-Dihydro-Avermectin-Bla-aglykon ($R_2$ = sek.$C_4H_9$) durch Reaktion mit 2-Nitrobenzolsulfonylchlorid eine 13β-Chlorierung durchführen lässt, wobei das 5-O-Silyl-13β-chlor-13-deoxi-22,23-dihydro-avermectin-Bla-aglykon ($R_2$ = sek.$C_4H_9$) gebildet wird.

Weitere Halogenierungen in der 13-Position sind bei Avermectinen nicht bekanntgeworden.

In der Milbemycin-Reihe sind direkte selektive Halogenierungen in der allylischen 13-Position nicht möglich.

Die vorliegende Erfindung betrifft nun Verfahren, die erlauben, in der 13-Position von Milbemycin- und 13-Deoxi-22,23-Dihydro-Avermectin-aglykon-Derivaten jedes der Halogene Fluor, Chlor, Brom, Jod sowie die OH-Gruppe gezielt einzuführen und damit zu hochwirksamen neuen Parasitiziden und Insektiziden der Formel I zu gelangen, die gleichzeitig auch für weitere Derivatisierungen verwendet werden können. Allen nachfolgenden Reaktionen ist der Einsatz von $\Delta^{13,14}$-15-Hydroxi-milbemycinen der Formel II als Reaktanden gemeinsam.

Ein Gegenstand vorliegender Erfindung ist somit ein Verfahren zur Herstellung von Verbindungen der Formel I, worin R in der β-Position steht und Fluor, Chlor, Brom oder Jod bedeutet, durch 13β-Halogenierung einer Verbindung der Formel II

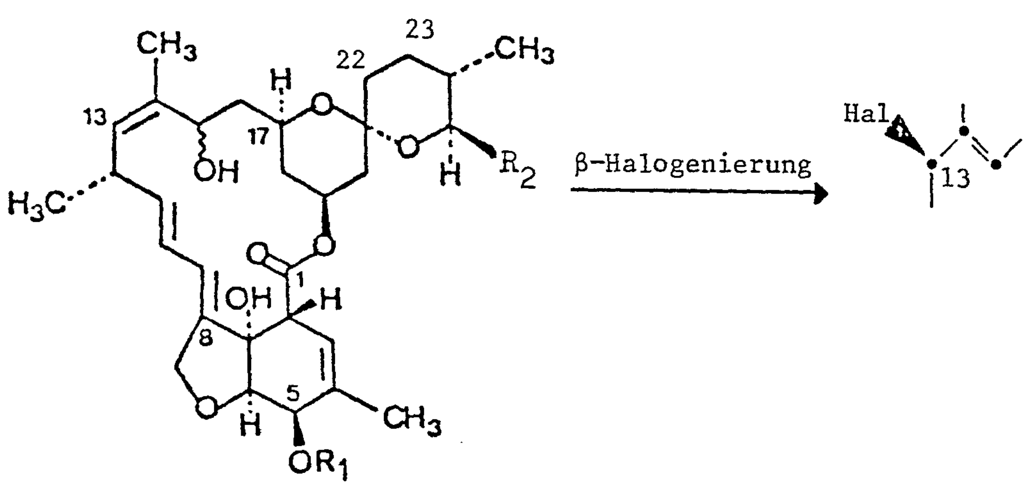


II

worin $R_1$ und $R_2$ die für Formel I gegebene Bedeutung haben, mit zur Einführung von Halogen in die 13β-Position geeigneten Halogenierungsmitteln. Vermutlich folgt dabei der anfänglichen Halogenierung der 15-OH-Gruppe überraschenderweise eine Allylumlagerung in die 13-Stellung unter Verschiebung der Doppelbindung in die 14,15-Position.

Für die vorstehende überraschende Reaktion werden wasserfreie und OH-gruppenfreie Lösungsmittel verwendet.

Sofern Verbindungen der Formel I mit $R_1$ = H gewünscht werden, muss die Halogenierungsreaktion mit einer Verbindung der Formel II durchgeführt werden, deren sehr reaktionsfähige 5-OH-Gruppe geschützt ist.

Als Schutzgruppen kommen die bereits für $R_1$ genannten Silyl- und Acyl-Gruppen in Frage oder z.B. ein Benzylether, Methoxiethoximethylether oder Dihydrofuran- oder Dihydropyran-Reste. Diese Schutzgruppen können in Verbindungen der Formel II oder bereits in einer früheren Reaktionsstufe eingeführt werden und können nach erfolgter Reaktion auf übliche Art wieder abgespalten werden.

Während, wie oben erwähnt, die aus "Tetrahedron Letters, Vol. 24, No. 48" bekanntgewordene Halogenierung von 22,23-Dihydro-avermectin-aglykon lediglich den nucleophilen Ersatz einer allylischen OH-Gruppe in 13α-Position durch 13β-Chlor betrifft, stellt die vorliegende selektive Halogenierung eines formal umgekehrt angeordneten Allyl-Alkohols ($\Delta^{13,14}$-15-ol) ein völlig verschiedenes Reaktionsprinzip dar, bei dem Halogen im Sinne einer stereospezifischen Allylumlagerung in die 13β-Position eintritt.

Als im obigen Sinne geeignete 13β-Fluorierungsmittel an Verbindungen der Formel II kommen $SF_4$ (Schwefeltetrafluorid) bei tiefen Temperaturen (-40°C oder darunter) oder $SeF_4$ (Selentetrafluorid) bei 0°-105°C, bevorzugt 5°-40°C, sowie ihre reaktionsanalogen Derivate in Frage. Zu diesen zählen bis-[Diethylamino]-schwefeldifluorid (=$[Et_2N]_2SF_2$), Diethylamino-dimethylaminoschwefeldifluorid (=$Me_2N-SF_2-NEt_2$), Piperidino-schwefeltrifluorid (=$N-SF_3$-Pip) Piperidino-selenotrifluorid (= $N-SeF_3$-Pip) und besonders das Dimethyl- und das Diethyl-aminoschwefeltrifluorid (=DAST = $Et_2N-SF_3$), das im Temperaturbereich von 0°C bis -100°C, bevorzugt -10°C bis -85°C, angewendet wird. Als geeignete inerte Lösungsmittel kommen

bei diesen Temperaturen flüssige Kohlenwasserstoffe wie Pentan, und Chlorkohlenwasserstoffatome in Frage, z.B. Dichlormethan. Der Einsatz von 13α-Hydroxi-Milbemycinen der Formel I führt unter den gleichen Bedingungen zu einem Gemisch von 13α-Fluor- und 13β-Fluormilbemycin-Derivaten der Formel I. Diese Reaktion ist ein weiterer Gegenstand vorliegender Erfindung.

Als im obigen Sinne geeignete 13β-Chlorierungsmittel bzw. 13β-Bromierungsmittel an Verbindungen der Formel II kommen Halogenierungsreagenzien wie Thionylchlorid ($SOCl_2$), Phosphortrichlorid ($PCl_3$), bzw. Thionylbromid ($SOBr_2$) und Phosphortribromid ($PBr_3$) oder eine Kombination von Triphenylphosphin und Brom in Frage. Die Reaktionstemperaturen liegen im Bereich von -40°C bis +10°C, bevorzugt -25° bis 0°C.

Als Lösungsmittel eignen sich die für die β-Fluorierung genannten Kohlenwasserstoffe und Chlorkohlenwasserstoffe. Es wird vorzugsweise in Gegenwart einer Base wie Triethylamin, Triethylendiamin, Pyridin oder anderen gearbeitet.

Als im obigen Sinne geeignete 13β-Jodierungsmittel an Verbindungen der Formel II kommen eine zweckmässig im Ueberschuss eingesetzte Kombination von Jod, Triphenylphosphin (=$PPh_3$) und Imidazol oder eine Kombination von Triphenylphosphin und 2,4,5-Trijodimidazol in Frage. Diese Reaktion lässt sich in Kohlenwasserstoffen, z.B. Hexan oder Toluol, oder in halogenierten Kohlenwasserstoffen wie Dichlormethan, Chloroform oder Chlorbenzol, bei -10°C bis +60°C, bevorzugt +10°C bis +40°C, durchführen.

Ein weiterer Gegenstand vorliegender Erfindung betrifft ein Verfahren zur Herstellung von Verbindungen der Formel I, worin R eine Hydroxi-Gruppe bedeutet, und es ist gekennzeichnet durch Reaktion einer Verbindung der Formel II mit Chromat-, Halochromat- oder Dichromat-Ionen, insbesondere mit Pyridiniumdichromat [=$(Pyr)_2^+Cr_2O_7$] oder mit Pyridiniumchlorochromat [=$(Pyr)^+ClCrO_3$].

Es werden inerte wasserfreie, vorzugsweise polare Lösungsmittel, z.B. Dimethylformamid (=DMF) verwendet. Die Reaktion wird bei Temperaturen von -10°C bis +60°C, bevorzugt +10°C bis +40°C, durchgeführt.

Es werden bei dieser Reaktion neben den gewünschten 13β-Hydroxi-Milbemycinen der Formel I auch 13-Oxo-Milbemycine gleicher Grundstruktur gebildet, (Formel IV), jedoch keine α-Hydroxi-Milbemycine. Die beiden so erhaltenen Reaktionsprodukte lassen sich durch übliche Trennungsmethoden, z.B. durch fraktionierte Kristallisation oder durch Chromatographie, voneinander trennen. Unter Chromatographie werden Säulen-, Dickschicht- oder Dünnschicht-Chromatographie an mineralischen Trägermaterialien wie Silicagel oder an organischen Austauscherharzen verstanden.

13-Oxo-Verbindungen der Formel IV

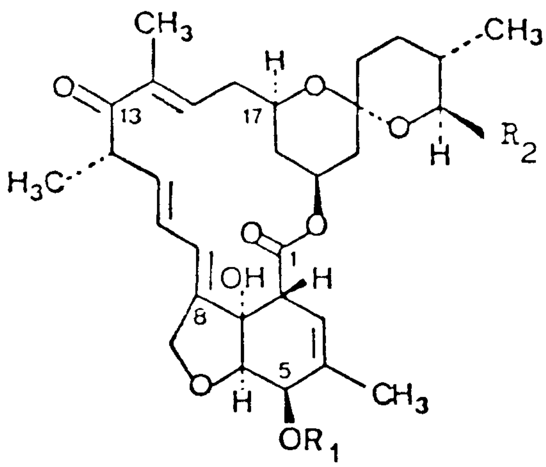

IV

worin $R_1$ und $R_2$ die für Formel I gegebene Bedeutung haben, stellen nun aber die wichtigen Zwischenprodukte zur Gewinnung der 13α-Hydroxi-Milbemycin-Derivate der Formel I mit $R_2 = CH_3$, $C_2H_5$ und iso$C_3H_7$ dar und die Verbindungen IV sind demgemäss ein weiterer Gegenstand vorliegender Erfindung.

Im Sinne eines erfindungsgemässen Folgeschritts werden aus Verbindungen der Formel IV durch selektive Hydrierung mit einem Metallhydrid, wie z.B. $NaBH_4$, in 13-Position nebeneinander 13α-Hydroxi-

und 13β-Hydroxi-Milbemycin-Derivate gebildet, die sich durch vorstehend erwähnte übliche physikalisch-chemische Trennungsmethoden auftrennen lassen. Aus 13α-Hydrox -Milbemycin-Derivaten der Formel I lassen sich mit den weiter oben für die 13β-Halogenierung beschriebenen Halogenierungsmitteln überraschenderweise 13α-Halogen-Milbemycin-Derivate der Formel I gewinnen.

Die 13-Oxo-Verbindungen der Formel IV lassen sich überraschenderweise gezielt aus den 13β-Hydroxy-Milbemycinen der Formel I mit Dialkylsulfiden oder Dialkylsulfoxiden, insbesondere in ihrer aktivierten Form, herstellen, wobei vorzugsweise in Gegenwart einer Base gearbeitet wird. Besonders geeignet sind Dimethylsulfid (DMS) und Dimethylsulfoxid (DMSO). Als aktivierte Form soll hier und im folgenden die Kombination aus Dialkylsulfoxid bzw. -sulfid und einem Aktivator dienen. Geeignete Aktivatoren sind zum Beispiel: Dicyclohexylcarbodiimid (DCC), Schwefeltrioxid-Pyridin (Pyridin•$SO_3$), N-Halogensuccinimide wie N-Chlorsuccinimid (NCS), Carbonsäureanhydride wie Essigsäureanhydrid und Trifluoressigsäureanhydrid, Thionylchlorid ($SOCl_2$), Sulfurylchlorid ($SO_2Cl_2$) sowie Oxalylchlorid. Manchmal ist der Zusatz von $CF_3COOH$ vorteilhaft. Als Basen werden z.B. organische Basen wie tertiäre Amine, insbesondere Trialkylamine wie Triethylamin und Pyridin eingesetzt. Als Lösungsmittel kommen die Dialkylsulfide und -sulfoxide selbst sowie aromatische Kohlenwasserstoffe wie Benzol, Toluol, Xylole und chlorierte Kohlenwasserstoffe, wie Methylenchlorid in Frage. Die Reaktionen wurden bei Temperaturen von -100°C und + 60°C, vorzugsweise -80°C bis +40°C durchgeführt. Einen Ueberblick über aktiviertes DMSO, DMS und günstige Reaktionsbedingungen gibt die nachfolgende Tabelle:

Tabelle Besonders bevorzugte Reaktionsbedingungen

| Agens | Aktivator | Lösungsmittel | Temperaturen | Katalysator/ Zusatz |
|---|---|---|---|---|
| DMSO | DCC | DMSO und/oder Toluol | 0° bis 50°C z.B. +25°C | Pyridin/ $CF_3COOH$ |
| DMSO | Pyridin•$SO_3$ | DMSO | 0° bis 50°C z.B. +22°C | $(C_2H_5)_3N$ |
| DMS | NCS | Toluol oder $CH_2Cl_2$ | -80° bis +30°C z.B. -25°C | $(C_2H_5)_3N$ |
| DMSO | $SOCl_2$ | $CH_2Cl_2$ | -80° bis +30°C z.B. -25°C | $(C_2H_5)_3N$ |
| DMSO | $SO_2Cl_2$ | $CH_2Cl_2$ | -80° bis +30°C z.B. -25°C | $(C_2H_5)_3N$ |
| DMSO | $(COCl)_2$ | $CH_2Cl_2$ | ± 0° bis +50°C z.B. -60°C | $(C_2H_5)_3N$ |
| DMSO | $(CH_3CO)_2O$ | DMSO | -100° bis +30°C z.B. +25°C | ——— |
| DMSO | $(CF_3CO)_2O$ | $CH_2Cl_2$ | -100° bis +30°C z.B. -60°C | $(C_2H_5)_3N$ |

Durch die Zugänglichkeit der Verbindungen der Formel II und ihre überraschenden Reaktionsmechanismen ist ein gangbarer Weg gefunden worden, um in der Stoffklasse der Milbemycine jedes der Halogenatome F, Cl, Br, J und die OH-Gruppe gezielt sowohl in die 13α- wie in die 13β-Stellung einzuführen.

Ein weiterer Gegenstand vorliegender Erfindung betrifft neue 13-substituierte Verbindungen der Formel I, worin

$R_1$ Wasserstoff, eine Silyl- oder Acylgruppe bedeutet,
und

a) R α-Hydroxi, Fluor, Chlor oder Jod ist, wenn
$R_2$ Methyl oder Ethyl bedeutet,
oder

b) R β-Hydroxi, α-Chlor, Fluor, Brom oder Jod ist, wenn $R_2$ Isopropyl oder sek.Butyl bedeutet,
oder

c) R β-Chlor ist, wenn $R_2$ Isopropyl bedeutet.

Die vorliegenden Verbindungen sollen als Verbindungen der Formel I* bezeichnet werden.

Unter diesen sind solche der Formel I* bevorzugt, bei denen $R_1$ Wasserstoff darstellt und

a') R α-Fluor, β-Fluor, α-Chlor, β-Chlor, α-Jod oder β-Jod ist, wenn $R_2$ Ethyl bedeutet, oder

b') R β-Hydroxi, α-Chlor, β-Chlor, α-Fluor, β-Fluor, α-Brom, β-Brom, α-Jod oder β-Jod ist, wenn $R_2$ Isopropyl bedeutet.

Ein weiterer Gegenstand vorliegender Erfindung betrifft Schädlingsbekämpfungsmittel gegen Ekto- und Endoparasiten sowie Schadinsekten, die neben üblichen Trägerstoffen und/oder Verteilungsmitteln als mindestens einen Wirkstoff eine Verbindung der Formel I enthalten.

Die Verbindungen der Formel II lassen sich aus 14,15-Epoxi-Milbemycinen der Formel III gewinnen, worin $R_1$ und $R_2$ die für die Formel I genannten Bedeutungen haben,

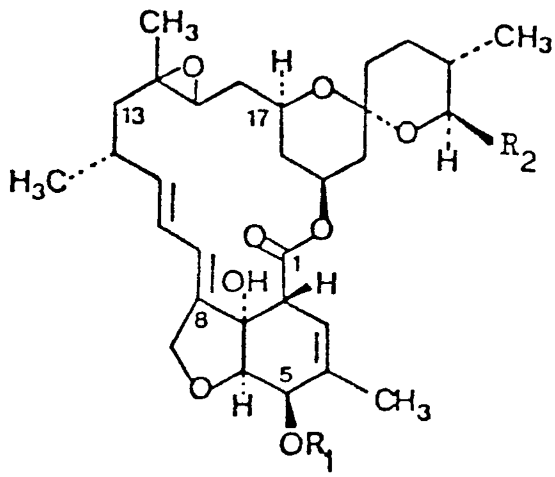

III

mit Hilfe des Komplex-Reagenzes $[HN_3]_m/Al(Ethyl)_3]_n$, worin m und n unabhängig die Zahl 1 oder 2 oder einen Zahlenwert zwischen 1 und 2 darstellen, in inerten trockenen Lösungsmitteln im Temperaturbereich von -30°C bis +10°C, vorzugsweise -20°C bis -5°C.

Als inerte Lösungsmittel kommen vorzugsweise aliphatische und aromatische Kohlenwasserstoffe wie Benzol, Toluol, Xylol, Petrolether; Ether wie Diethylether, tert.Butylmethylether, Tetrahydrofuran, Dioxan, Anisol in Frage.

Die Reaktion wird vorteilhaft unter Schutzgas, wie Stickstoff oder Argon durchgeführt.

Stickstoffwasserstoffsäure $HN_3$ lässt sich in statu nascendi in den $[HN_3]_m/[Al(Et)_3]_n$-Komplex überführen, indem man im vorgesehenen trockenen Lösungsmittel oder Lösungsmittel-Gemisch Na-Azid suspendiert und daraus mit einer stärkeren Säure, z.B. $H_2SO_4$ (bevorzugt Oleum, um absolut trockene Reaktionsbedingungen zu gewährleisten), $HN_3$ in der Lösung in Freiheit setzt. $Al(Et)_3$ sollte in der Lösung bereits vorliegen oder kurz danach zugegeben werden. Die zur Reaktion vorgesehene Epoxi-Verbindung kann gleichfalls bereits vorliegen oder zu einem geeigneten Zeitpunkt zur Lösung dosiert werden.

Die für die Verbindungen II verwendeten Ausgangsverbindungen der Formel III lassen sich leicht herstellen durch Epoxidierung der aus der US-PS 3,950,360 bekanntgewordenen und ursprünglich als "Antibiotika B-41-A", später als "Milbemycin-A"-Verbindungen und der aus der US-PS 4,346,171 bekanntgewordenen und als "B-41-D" oder "Milbemycin-D" bezeichneten Verbindungen der Formel sowie der aus der US-PS 4,173,571 bekanntgewordenen 13-Deoxi-22,23-dihydro-Avermectine ($R_2$ = sec.Butyl) der Formel

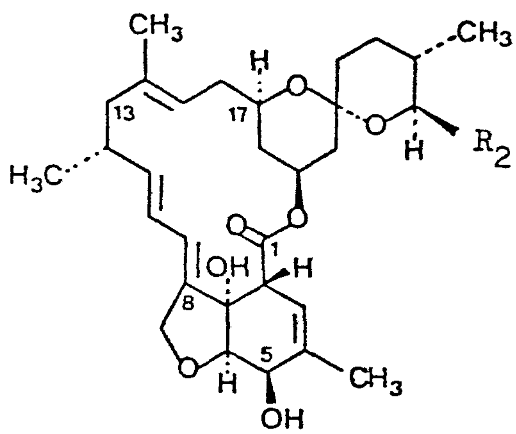

$R_2 = CH_3$ Milbemycin $A_3$
$R_2 = C_2H_5$ Milbemycin $A_4$
$R_2 = isoC_3H_7$ Milbemycin D
$R_2 = sec.C_4H_9$ 13-Deoxi-22,23-dihydro-C-076-Bla-aglycon.

Die Epoxidierung wird in einer Lösungsmittelphase im Temperaturbereich von -10°C bis +20°C, vorzugsweise -5°C bis +5°C, durchgeführt.

Zur Epoxidierung kommen Persäuren wie Peressigsäure, Trifluorperessigsäure, Perbenzoesäure, Chlorperbenzoesäure und andere in Frage.

Durch übliche Acylierung der 5-OH-Gruppe mit den entsprechenden Acylhalogeniden oder Acylanhydriden oder durch Reaktion der 5-OH-Gruppe mit dem entsprechend substituierten Silan-Derivat der Formel

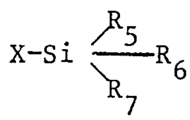

werden alle jene vorgenannten Derivate der Formeln I bis IV hergestellt, bei denen $R_1$ eine andere Bedeutung als Wasserstoff hat, und worin $R_5$, $R_6$ und $R_7$ die für die Formel I genannten Reste darstellen, wobei der Begriff Acylhalogenid für Acylchlorid oder Acylbromid steht und wobei X eine Silylabgangsgruppe bedeutet. Zu den Silylabgangsgruppen X zählen beispielsweise Bromid, Chlorid, Cyanid, Azid, Acetamid, Trifluoracetat, Trifluormethansulfonat. Diese Aufzählung stellt keine Limitierung dar, der Fachmann kennt weitere typische Silylabgangsgruppen.

5-O-Acylierungen und 5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0°C bis 80°C, bevorzugt bei 10°C bis 40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silyl- und Acylreste $R_1$ in der 5-Position geschieht durch selektive milde Hydrolyse ($\longrightarrow$ R=H) mit z.B. Arylsulfonsäure in alkoholischer Lösung oder nach einer anderen dem Fachmann geläufigen Methode.

Die Verbindungen der Formeln I eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae,

Sarcoptidae, Psoroptidae; die Ordnungen Mallophaga; Siphonaptera, Anoplura (z.B. Familie der Haematopinidae); unter der Ordnung Diptera insbesondere Schädlinge der Familien Muscidae, Calliphoridae, Oesterridae, Tabanidae, Hippoboscidae, Gastrophilidae.

Die Verbindungen I sind auch einsetzbar gegen Hygiene-Schädlinge insbesondere der Ordnungen Diptera mit den Familien Sarcophagidae, Anophilidae, Culicidae; der Ordnung Orthoptera, der Ordung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnungen Homoptera, insbesondere gegen Schädlinge der Familien Aphididae, Delphacidae, Cicadellidae, Psyllidae, Loccidae, Diaspididae und Eriophyidae (z.B. die Rostmilbe auf Citrusfrüchten); der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizide gegen Schädlinge im Erdboden geeignet.

Die Verbindungen der Formeln I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen I sind auch wirksam gegen Pflanzen-Nematoden der Arten Meloidogyne, Heterodera, Pratylenchus, Ditylenchus, Radopholus, Rhizoglyphus und andere.

Darüberhinaus sind die Verbindungen I gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesphagostomum, Chabertia, Trichuris, Strongylus, Trichonema, Dictyocaulus, Cappillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Die Verbindungen I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend ausgewählt.

Die Verbindungen I werden bei Warmblütern in Aufwandmengen von 0,01 bis 50 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen,

wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation- und/oder anionaktive Tenside mit gutem Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.
Die in der Formulierungstechnik gebräuchlichen Tenside sind u.a. in folgenden Publikationen beschrieben:

"Mc Cutcheon's Detergents and Emulsifiers Annual" MC Publishing Corp., Ringwood, New Jersey, 1982.

Die pestiziden Zubereitungen enthalten in der Regel 0,01 bis 95 %, insbesondere 0,1 bis 80 %, Wirkstoff, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die Verbindungen der Formel stellen auch vielseitig reaktionsfähige Produkte zur Gewinnung weiterer Milbemycin-Derivate dar.

## Herstellungsbeispiele:

## Herstellung von Ausgangs- und Zwischenprodukten

### 1. Herstellung von wasserfreiem $HN_3$

1.1. In Benzol. Die Herstellung erfolgte nach der Vorschrift von H. Wolf, Org. Reactions 3, 307 (1946). Die dabei erhaltene Lösung von $HN_3$ in Benzol wurde zweimal über wasserfreiem $Na_2SO_4$ (30 Min. auf einer Flamme erhitzt und im Exsikkator abgekühlt) getrocknet und bei 0°C ⟶ 5°C durch Watte abfiltriert. Die Lösung wurde bei 4°C im Kühlschrank aufbewahrt.

1.2. In Ether. Die Herstellung erfolgte analog zur obigen Vorschrift, ausser dass bei der Zugabe der Schwefelsäure die Reaktionstemperatur zwischen -20°C und -10°C gehalten wurde; nach beendeter $H_2SO_4$ Zugabe wurde das Reaktionsgemisch auf -5°C erwärmt.

### 2. Herstellung von 14,15-Epoxi-Milbemycin D (Formel III)

Zu einer Lösung von 550 mg Milbemycin D in 5 ml Dichlormethan wurde unter Eiskühlung eine Lösung von 170 mg Chlorperbenzoesäure in 5 ml Dichlormethan gegeben. Nach 1-stündigem Rühren bei 0° bis 5°C wurden nochmals 170 mg des Oxidationsmittels hinzugefügt und weitere 30 min. gerührt. Nach beendeter Reaktion wurde die Lösung in eine eisgekühlte Lösung von Natriumsulfit gegossen und mit Essigsäureethylester extrahiert. Die vereinigten Extrakte wurden einmal mit Wasser gewaschen, getrocknet und im Vakuum eingedampft. Das Rohprodukt wurde durch Chromatographierung über eine Silicagel-Säule (Elutionsmittel n-Hexan/Essigsäureethylester 20:15) gereinigt. Es wurden 450 mg 14,15-Epoxi-Milbemycin D als amorphe, weisse Substanz erhalten.

### 3. Herstellung von 15-Hydroxi-$\Delta^{13,14}$-Milbemycin D (Formel II)

Es wurden bei -20°C zu einer Lösung von 2,1 ml (1,75 g, 15,3 mmol)

Triethylaluminium in 8,5 ml abs. Diethylether 8.4 ml (0,41 g, 9,53 mmol) einer 6,96 proz. Lösung von $HN_3$ in Diethyl-Ether gegeben, die dann bei -10°C unter stark exothermer Reaktion zu 1,8 g (3,15 mmol) 14,15-Epoxi-Milbemycin D (in Substanz) gegeben wurde. Nach 1 Stunde bei Raumtemperatur wurden 4 ml absoluter Ether zugegeben und das gallertige Reaktionsgemisch wurde kräftig gerührt. Nach 4 Stunden wurde wie in Vorschrift 2 aufgearbeitet und die Chromatographie an 70 g Kieselgel ($CH_2Cl_2$/Aceton 10:1) ergab 200 mg (10%) 14-Azido-15-hydroxi-Milbemycin D und 820 mg (45%) 15-Hydroxy-$\Delta^{13,14}$-Milbemycin D, Smp.: 151°C-153°C (aus Methanol).

4. Herstellung von 5-O-t-Butyldimethylsilyl-14,15-epoxi-Milbemycin D

Eine Lösung von 2,21 g (3,86 mmol) 14,15-Epoxi-Milbemycin D 757 mg (5,02 mmol) t-Butyl dimethylchlorsilan und 342 mg (5,02 mmol) Imidazol in 4 ml DMF wurde 90 min. bei Raumtemperatur gerührt. Anschliessend wurden 80 ml Diethylether zugegeben, und das Gemisch wurde über 20 g Kieselgel filtriert und eingeengt. Es wurden 2,65 g (100%) 5-O-t-Butyldimethylsilyl-14,15-epoxi-Milbemycin D erhalten.

$^1$H-NMR (300MHz. Lösungsmittel $CDCl_3$. Messwerte $\delta$ bezogen auf $Si(CH_3)_4$ = TMS).

0,12 ppm (s) $(CH_3)_2Si-O-$;

0,92 ppm (s) (t.-$C_4H_9$)Si-O-;

1,23 ppm (breites s) ($C_{14}CH_3$, d.h. Signal der $CH_3$-Gruppe in 14-Position);

2,56 ppm (d; J = 9) ($C_{15}H$, d.h. Signal des Protons in 15-Position).

Aehnlich lässt sich durch Reaktion mit Trimethylsilyl-trifluormethansulfonat das entsprechende 5-O-Trimethylsilyl-14,15-epoxi-Milbemycin D herstellen, Smp. 92-97°C.

5. Herstellung von 5-O-t-Butyldimethylsilyl-15-hydroxi-$\Delta^{13,14}$-Milbemycin D (Formel II)

Eine Lösung des $HN_3$/$Et_3Al$ - Komplex-Reagenz (hergestellt aus einer Lösung von 4,97 ml Triethylaluminium in 7 ml abs. Tetrahydrofuran (THF) und 9,15 ml einer 2,39 M Lösung von $HN_3$ (21,9 mmol) in abs. Diethylether wurde unter Argon zu einer Lösung von 5.0 g (7,29 mmol) 5-O-Butyldimethylsilyl-14,15-epoxi-Milbemycin D in ca. 20 ml abs. THF gegeben, und das Gemisch wurde 16 Std. unter Rückfluss erhitzt. Anschliessend wurden bei Raumtemperatur 250 ml Ether, 2 ml Methanol und schliesslich ein Gemisch von 10 g $Na_2SO_4 \cdot 10\ H_2O$ und 10 g Celite zugegeben. Das Gemisch wurde filtriert, eingeengt, und die Chromatographie des Rohproduktes an 160 g Kieselgel (0 - 30% Essigsäureethylester in Hexan) ergab 2,37 g (47%) 5-O-t-Butyldimethylsilyl-15-hydroxi-$\Delta^{13,14}$-Milbemycin D.

$^1$H-NMR (300 MHz, $CDCl_3$):
1,59 (d; J = 1) ($C_{14}CH_3$);
4,06 (dd; $J_1$ = 11; $J_2$ = 4) ($C_{15}H$);
5,15 (d; J = 8) ($C_{13}H$).

Daneben wurden 109 mg (2%) 13β-Azido-5-O-t-butyldimethylsilyl-Milbemycin D gewonnen.

6. Herstellung von 14,15-Epoxi-Milbemycin $A_4$ (Formel III) ($R_2$=$C_2H_5$)

Zu einer Lösung von 5,7 g (10,5 mmol) Milbemycin $A_4$ in 140 ml Dichlormethan und 120 ml 0,5 M $NaHCO_3$-Lösung wurde bei Raumtemperatur eine Lösung von 2,43 g (14,08 mmol) m-Chlorobenzopersäure in 70 ml Dichlormethan tropfenweise zugegeben. Das Gemisch wurde 1 Std. bei Raumtemperatur kräftig gerührt und dann mit 300 ml Dichlormethan verdünnt. Die organische Phase wurde mit wässriger $NaHCO_3$-Lösung gewaschen, mit $Na_2SO_4$ getrocknet und eingeengt. Es wurden 5,7 g Epoxid als Rohprodukt erhalten.

7. Herstellung von 5-O-t-Butyldimethylsilyl-14,15-epoxi-Milbemycin $A_4$ (Formel III)

5,7 g 14,15-Epoxi-Milbemycin $A_4$ wurden in 10 ml trockenem Dimethylformamid (DMF) gelöst. Bei Raumtemperatur wurden 0,63 g (9,16 mmol) Imidazol und 1,4 g (9,34 mmol) t-Butyldimethylchlorsilan zugegeben. Das Gemisch wurde 1 Std. bei Raumtemperatur gerührt und an 150 g Kieselgel chromatographiert (Hexan/Ether 4:1), wobei 2,84 g (40% d.Th. Ausbeute, bezogen auf Milbemycin $A_4$) des silylierten Epoxi-Derivats erhalten wurden.

8. Herstellung von 5-O-t-Butyldimethylsilyl-15-hydroxi-$\Delta^{13,14}$-Milbemycin $A_4$ (Formel II)

Das Komplex-Reagenz $HN_3$/Al (Ethyl)$_3$ wurde wie folgt hergestellt: 2,8 ml (12,2 mmol) $Al(C_2H_5)_3$ in 4 ml abs. THF wurden unter Argon bei ca. -20°C langsam mit 5,28 ml (20,4 mmol) einer 10%igen Lösung von $HN_3$ in abs. Diethylether versetzt. Zu dieser Lösung wurde unter Argon eine Lösung von 2,84 g (4,25 mmol) der im Beispiel 7 erhaltenen Verbindung gegeben, und das so erhaltene Gemisch wurde 4 Std. unter Rückfluss erhitzt. Bei Raumtemperatur wurden 500 ml Diethyl-Ether , 10 g $Na_2SO_4 \cdot 10H_2O$ und 10 g Celite zugegeben, und das Gemisch wurde filtriert und eingeengt. Die Chromatographie des Rohproduktes an 100 g Kieselgel (Hexan/Diethylether 7:2) ergab 1,72 g (60% d.Th.) der Titel-Verbindung.

$^1$H-NMR (300 MHz, $CDCl_3$):
1,59 (br. s) ($C_{14}CH_3$);
4,05 (br. s) ($C_{15}H$);
5,15 (d; J = 6) ($C_{13}H$).

Daneben wurden 0,1 g 13β-Azido-5-O-t-butyldimethylsilyl-Milbemycin $A_4$ erhalten.

9. Herstellung von 15-Hydroxi-$\Delta^{13,14}$-Milbemycin $A_4$ (Formel II)

Die Hydrolyse der im Beispiel 8 genannten Titelverbindung mit einer 1%ig.-Lösung von p-Toluolsulfonsäure in Methanol und die Aufarbeitung in Diethylether mit 5%ig. Na-Hydrogencarbonat-Lösung ergab die Titel-Verbindung.

10. Herstellung von 14,15-Epoxi-Milbemycin $A_3$ (Formel III) ($R_2$ = $CH_3$)

Nach der Vorschrift des Beispiels 2 werden aus 220 mg Milbemycin $A_3$ in 5 ml Dichlormethanund 320 mg Benzopersäure in 5 ml Dichlormethan bei -2° bis +5°C während 1,5 Std. und Reinigung über eine Silicagel-Säule 190 mg 14,15-Epoxi-Milbemycin $A_3$ gewonnen.

11. Herstellung von 5-O-Methyldiphenylsilyl-14,15-epoxi-Milbemycin $A_3$

Nach der Vorschrift des Beispiels 4 werden aus 190 mg 14,15-Epoxi-Milbemycin $A_3$ und 120 mg Diphenylmethylchlorsilan in Gegenwart von Imidazol 217 mg der Titel-Verbindung erhalten.

12. Herstellung von 5-O-Methyldiphenylsilyl-15-hydroxi-$\Delta^{13,14}$-Milbemycin $A_3$ (Formel II)

Analog zur Epoxid-Spaltung des Beispiels 5 werden aus 210 mg 5-O-Methyldiphenylsilyl-14,15-epoxi-Milbemycin $A_3$ in absolutem Diethylether mit Hilfe des Komplex-Reagenz $HN_3/Et_3Al$ unter Argon nach anschliessender Reinigung 203 mg der Titelverbindung erhalten.

$^1$H-NMR (300 HMz, $CDCl_3$);

1,58 (br. s)($C_{14}CH_3$);

4,05 (br. s)($C_{15}H$);

5,15 (d; J=6)($C_{13}H$).

13. Herstellung von 15-Hydroxi-$\Delta^{13,14}$-Milbemycin $A_3$

Analog zu Beispiel 2 wird das Reagenz $HN_3/Al(C_2H_5)_3$ frisch hergestellt und bei -10°C zu einer Lösung von 830 mg (3,05 mmol) 14,15-Epoxi-Milbemycin $A_3$ in 7 ml trockenem Diethylether getropft. Nach der Aufarbeitung werden 385 mg 15-Hydroxi-$\Delta^{13,14}$-Milbemycin $A_3$ und 92 mg 14-Azido-15-hydroxi-Milbemycin $A_3$ erhalten.

14. Herstellung von 13-Deoxi-14,15-epoxi-22,23-dihydro-avermectin-Bla-aglykon ($R_2$ = sec.$C_4H_9$) (Formel III)

Analog zu Beispiel 6 erhält man aus 520 mg 13-Deoxi-22,23-dihydro-avermectin-Bla-aglykon [Tetrahedron Letters, Vol. 24, Nol 48, pp. 5333-5336 (1983)] und 210 mg m-Chlorbenzopersäure in 20 ml Dichlormethan 510 mg der Titelverbindung.

15. Herstellung von 5-O-t.Butyldimethylsilyl-13-deoxi-14,15-epoxi 22,23-dihydro-avermectin-Bla-aglykon (Formel III)

Analog zu Beispiel 7 erhält man aus 220 mg der Titelverbindung von Beispiel 14 und 55 mg tert.Butyldimethylchlorsilan in Gegenwart von 25 mg Imidazol in 5 ml trockenem DMF 108 mg der Titelverbindung.

16. Herstellung von 13-Deoxi-15-hydroxi-$\Delta^{13,14}$-22,23-dihydro-avermectin-Bla-aglykon (Formel II)

Analog zu Beispiel 3 erhält man aus 220 mg der Titelverbindung von Beispiel 15 mit dem Komplex-Reagenz, bestehend aus 320 mg $Al(C_2H_5)_3$ und 110 mg einer 6,96%igen Lösung von $HN_3$ in total 16 ml trockenem Diethylether 112 mg der Titelverbindung. Daneben werden 61 mg 13-Deoxi-14-azido-15-hydroxi-22,23-dihydro-avermectin-Bla-aglykon erhalten.

17. Herstellung von 13-Oxo-Milbemycin D (Formel IV)

Zu einer Lösung von 53 mg (0,077 mmol) 5-O-tert.-Butyldimethylsilyl-13-oxo-Milbemycin D in 1 ml Methanol werden 1 ml einer 2%igen Lösung von p-Toluolsulfonsäure in Methanol gegeben. Nach 1 h Rühren bei Raumtemperatur über 5 g Kieselgel gefiltert, eingeengt und das erhaltene Rohprodukt an 10 g Kieselgel mit Essigester/Hexan 1:1 chromatographiert. Man erhält 31 mg (70 %) 13-Oxo-Milbemycin D.

$^1$H-NMR (250 MHz, $CDCl_3$):
3,04 ( bt, J = 8) ($C_{25}H$)
4,30 (dt, $J_d$ = 1, $J_t$ = 7) ($C_5H$)
4,67 (dd, $J_1$ = 15, $J_2$ = 2) $C_{27}\underline{H}H$)
4,74 (dd, $J_1$ = 15, $J_2$ = 2) ($C_{27}H\underline{H}$)
5,86 (dt, $J_d$ = 12, $J_t$ = 2,5) ($C_9H$)
6,05 (dd, $J_1$ = 12, $J_2$ = 15) ($C_{10}H$)
6,24 (t, J = 9) ($C_{15}H$)

18. Herstellung von 5-O-tert.-Butyldimethylsilyl-13-oxo-Milbemycin D (Formel IV)

Eine Lösung von 280 µl (3,26 mmol) Oxalylchlorid in 5 ml Methylenchlorid wird bei -60°C mit 460 µl (6,48 mmol) DMSO versetzt. Nach 5 Min. wird eine Lösung von 1,11 g (1,62 mmol) 5-O-tert.-Butyldimethylsilyl-13β-hydroxy-Milbemycin D in 5 ml Methylenchlorid zugetropft. Nach 30 Min. bei -60°C bis -40°C werden 2,3 ml (16,5 mmol) Triethylamin zugegeben. Das Reaktionsgemisch lässt man auf 20°C erwärmen, filtert an 5 g Kieselgel und engt die Lösung ein. Die Chromatographie des Rohprodukts an 50 g Kieselgel (Aether/Hexan 1:2) ergab 0,99 g (90 %) 5-O-tert.-Butyldimethylsilyl-13-oxo-Milbemycin D.

$^1$H-NMR (250 MHz; $CDCl_3$):
3,04 (bd, J = 8) ($C_{25}H$)
4,43 (bd, J = 6) ($C_5H$)
4,60 (dd, $J_1$ = 15, $J_2$ = 3) ($C_{27}\underline{H}H$)
4,72 (dd, $J_1$ = 15, $J_2$ = 2) ($C_{27}H\underline{H}$)
5,82 (bd, J = 12) ($C_9H$)
6,04 (dd, $J_1$ = 15, $J_2$ = 12) ($C_{10}H$)
6,23 (t, J = 9) ($C_{15}H$)

19. Herstellung von 5-O-tert.-Butyldimethylsilyl-13-oxo-Milbemycin $A_4$ (Formel IV)

Analog Beispiel 18 werden aus 470 mg (0,69 mmol) 5-O-tert.-Butyldimethylsilyl-13β-hydroxy-Milbemycin $A_4$ und 118 µl (1,37 mmol) Oxalylchlorid, 195 µl (2,75 mmol) DMSO und 0,96 ml (6,88 mmol) Triethylamin 412 mg (88 %) 5-O-tert.-Butyldimethylsilyl-13-oxo-Milbemycin $A_4$ erhalten.

$^1$H-NMR (250 MHz; $CDCl_3$):
3,05 (dt, $J_d$ = 2, $J_t$ = 9) ($C_{25}H$)
4,45 (bd, J = 6) ($C_5H$)
4,61 (dd, $J_1$ = 15, $J_2$ = 3) ($C_{27}\underline{H}H$)
4,75 (dd, $J_1$ = 15, $J_2$ = 3) ($C_{27}H\underline{H}$)
5,83 (bd, J = 12) ($C_9H$)
6,02 (dd, $J_1$ = 15, $J_2$ = 12) ($C_{10}H$)
6,22 (t, J = 9) ($C_{15}H$)

20. Herstellung von 13-Oxo-Milbemycin $A_4$ (Formel IV)

Eine Lösung von 54 mg (0,081 mmol) 5-O-tert.-Butyldimethylsilyl-13-oxo-Milbemycin $A_4$ in 1 ml 40 % HF/Acetonitril (5:95) wird 1 Stunde bei Raumtemperatur gerührt, über Kieselgel filtriert und eingeengt. Die Chromatographie an 10 g Kieselgel mit Essigester/Hexan (1:1) ergibt 35 mg (78 %) 13-Oxo-Milbemycin $A_4$

$^1$H-NMR (250 MHz, $CDCl_3$):
3,03 (dt, $J_d$ = 3, $J_t$ = 10) ($C_{25}H$)
4,31 (dt, $J_d$ = 2, $J_t$ = 7) ($C_5H$)
4,68 (dd, $J_1$ = 15, $J_2$ = 2) ($C_{27}\underline{H}H$)
4,74 (dd, $J_1$ = 15, $J_2$ = 2) ($C_{27}H\underline{H}$)
5,85 (dt, $J_d$ = 12, $J_t$ = 2) ($C_9H$)
6,03 (dd, $J_1$ = 15, $J_2$ = 12) ($C_{10}H$)
6,21 (t, J = 9) ($C_{15}H$)

21. Herstellung von 5-O-tert.Butyldimethylsilyl-13-oxo-Milbemycin D

Eine Lösung von 158 mg (0,23 mmol) 5-O-tert.Butyldimethylsilyl-15-hydroxi-$\Delta^{13,14}$-milbemycin D und 500 mg (1,33 mmol) PDC in 1 ml DMF wurden 40 min. bei Raumtemperatur gerührt. Das Gemisch wurde mit 20 ml Dichlormethan verdünnt, durch 5 g Kieselgel filtriert und eingeengt. Die Chromatographie des Rohprodukts an 20 g Kieselgel (Ethylacetat/Hexan 1:4) ergab 67 mg 5-O-t-Butyldimethylsilyl-13-oxo-Milbemycin D und zeigte die in Beispiel 18 genannten NMR-Spektren.

Herstellung von Verbindungen der Formel I

**22. Herstellung von 5-O-tert.-Butyldimethylsilyl-13α-hydroxy-Milbemycin $A_4$ (Formel I)**

Zu einer Lösung von 70 mg (0,104 mmol) 5-O-tert.-Butyldimethylsilyl-13-oxo-Milbemycin $A_4$ in 1 ml Ethanol werden 0,55 ml (0,11 mmol) einer 0,2 M Lösung von Natriumborhydrid in Ethanol gegeben. Nach 30 Min. Rühren bei Raumtemperatur werden 1 ml gesättigter $NH_4Cl$-Lösung zugegeben. Die resultierende Lösung wird über Kieselgel filtriert und eingeengt. Die Chromatographie des Rohprodukts an Kieselgel (Essigester/Hexan 1:6) liefert 55 mg (78 %) 5-O-tert.-Butyldimethylsilyl-13α-hydroxy-Milbemycin $A_4$.

$^1$H-NMR (250 MHz, $CDCl_3$):
3,00 (dt, $J_d$ = 2, $J_t$ = 9) ($C_{25}H$)
3,89 (bs) ($C_{13}H$)
4,32 (bd, J = 5) ($C_5H$)
4,45 (dd, $J_1$ = 15, $J_2$ = 2) ($C_{27}\underline{H}H$)
4,57 (dd, $J_1$ = 15, $J_2$ = 2) ($C_{27}H\underline{H}$)

23. Herstellung von 13α-Hydroxy-Milbemycin $A_4$ (Formel I)

Eine Lösung von 20 mg (0,030 mmol) 5-O-tert.-Butyldimethylsilyl-13α-hydroxy-Milbemycin $A_4$ in 1 ml 40 % HF/Acetonitril (5:95) wird 1 Stunde bei Raumtemperatur gerührt, über Kieselgel filtriert und eingeengt. Die Chromatographie des Rohproduktes an 5 g Kieselgel (Essigester/Hexan 1:1) ergibt 14 mg (84 %) 13α-Hydroxy-Milbemycin $A_4$.

$^1$H-NMR (250 MHz, $CDCl_3$):
3,09 (dt, $J_d$ = 2, $J_t$ = 9) ($C_{25}H$)
4,02 (bs) ($C_{13}H$)
4,31 (bt, J = 7,5) ($C_5H$)
4,66 (dd, $J_1$ = 15, $J_2$ = 2) ($C_{27}\underline{H}H$)
4,74 (dd, $J_1$ = 15, $J_2$ = 2) ($C_{27}H\underline{H}$)

24. Herstellung von 5-O-tert.Butyldimethylsilyl-13β-fluoro-milbemycin D und von 13β-Fluoro-Milbemycin D

Eine Lösung von 18,3 µl (24 mg; 0,15 mmol) Diethylaminoschwefeltrifluorid (DAST) in 0,15 ml trockenem Dichlormethan wurde bei -60°C unter Argon zu einer Lösung von 103 mg (0,15 mmol) 5-O-tert. Butyldimethylsilyl-15-hydroxi-$\Delta^{13,14}$-Milbemycin D in 1,5 ml Dichlormethan getropft. Nach 10 min. wurde mit 5%iger wässriger $NaHCO_3$-Lösung und Diethylether aufgearbeitet. Die organische Phase wurde mit $MgSO_4$ getrocknet und eingeengt. Man erhielt 100 mg 5-O-tert.Butyldimethylsilyl-13β-fluoro-milbemycin D, das mit 2 ml einer 1%igen Lösung von p-Toluolsulfonsäure in Methanol 1 Std. bei Raumtemperatur gerührt und dann mit 5%iger wässriger $NaHCO_3$-Lösung und durch Ausschütteln mit dreimal 2 ml Diethylether aufgearbeitet

wurde. Die Chromatographie des Rohprodukts an 20 g Kieselgel (Laufmittel Aceton/Dichlormethan 1:12) ergab 58 mg (67% d.Th.) 13β-Fluoro-Milbemycin D.

$^{1}$H-NMR (300 MHz; $CDCl_3$; TMS):
1,61 ppm (br. s)($C_{14}CH_3$)
2,5-2.7 ppm (m)($C_{12}H$)
4,40 ppm (dd; $J_1$=48; $J_2$=10)($C_{13}H$).

Massenspektrum m/e: 574 ($M^+$ , 6% von $C_{33}H_{47}FO_7$), 446, 374, 332, 428, 151.

25. Herstellung von 5-O-tert.Butyldimethylsilyl-13β-fluoro-milbemycin $A_4$ und von 13β-Fluoromilbemycin $A_4$.

Analog zu Beispiel 24 erhält man aus 202 mg 5-O-tert.Butyldimethylsilyl-15-hydroxi-$\Delta^{13,14}$-milbemycin $A_4$ und 36,2 µl DAST 183 mg 5-O-tert.Butyldimethylsilyl-13β-fluoromilbemycin $A_4$ und durch Entsilylierung mit methanolischer p-Toluolsulfonsäure 114 mg 13β-Fluoromilbemycin $A_4$.

$^{1}$H-NMR (300 MHz; $CDCl_3$; TMS):
1,61 (s)($C_{14}CH_3$)
4,42 (dd; $J_1$=47; $J_2$= 10)($C_{13}H$).

26. Herstellung von 5-O-Methyldiphenylsilyl-13β-fluoro-milbemycin $A_3$ und von 13-β-Fluoro-milbemycin $A_3$.

Analog zu Beispiel 24 erhält man aus 108 mg 5-O-Methyldiphenylsilyl-15-hydroxi-$\Delta^{13,14}$-Milbemycin $A_3$ und 19,0 µl DAST 81 mg 5-O-Methyldiphenylsilyl-13β-fluoro-milbemycin $A_3$ und durch Entsilylierung mit methanolischer p-Toluolsulfonsäure in 5-Position 68 mg 13β-Fluoromilbemycin $A_3$.

27. Herstellung von 5-O-tert.Butyldimethylsilyl-13β-chlor-milbemycin D und von 13β-Chlor-milbemycin D

Zu einer Lösung von 50 mg (0,073 mmol) 5-O-tert.Butyldimethylsilyl-15-hydroxi-$\Delta^{13,14}$-Milbemycin D in 1,5 ml trockenem Dichlormethan wurden unter Argon und bei Eis/Methanol-Kühlung (ca. -10°C) 8,5 μl (13,9 mg, 0,116 mmol) Thionylchlorid getropft. Die Reaktion wird begünstigt durch die Gegenwart von 2 Aequivalenten (0,146 mmol) Triethylamin. Nach 5 min. wurde mit 5%iger wässriger $NaHCO_3$-Lösung und dann mit Diethylether ausgeschüttelt. Die organische Phase wurde mit $MgSO_4$ getrocknet und eingeengt. Die Säulenchromatographie des Rohprodukts an 20 g Kieselgel (Laufmittel Ethylacetat/Hexan 1:20) lieferte 17 mg (30% d.Th.) 5-O-tert.Butyldimethylsilyl-13β-chlor-milbemycin D.

$^1$H-NMR (300 MHz; $CDCl_3$; TMS):
0,93 (s)($C_5$-$OSiC(CH_3)_3$-)
0,13 (s)($C_5$-$OSi(CH_3)_2$-)
2,45-2,65(m)($C_{12}H$)
4,08 (d; J=11)($C_{13}H$).

Die Behandlung der silylierten Verbindung mit 1%iger p-Toluolsulfonsäure in Methanol während 1 Stunde lieferte nach Behandeln mit 5%iger wässriger $NaHCO_3$-Lösung, Aufnahme in Diethylether und Chromatographie an Kieselgel (Laufmittel Ethylacetat/Hexan 2:3) in quantitativer Ausbeute 13β-Chlormilbemycin-D.

$^1$H-NMR (300 MHz; $CDCl_3$; TMS):
1,67 (s)($C_{14}CH_3$);
5,24 (d; J=11)($C_{13}$ αH).

28. Herstellung von 5-O-tert.Butyldimethylsilyl-13β-brom-milbemycin D und von 13β-Brom-milbemycin D

Zu einer Lösung von 182 mg (0,265 mmol) 5-O-tert.Butyldimethylsilyl-15-hydroxi-$\Delta^{13,14}$-milbemycin D in 5 ml trockenem Dichlormethan wurden unter Argon bei -10°C 0,013 ml (36 mg, 0,133 mmol) $PBr_3$ unter Rühren zugetropft. Nach 5 min. wurde 0,1 ml Methanol zugegeben, mit Ether verdünnt, und die organische Phase wurde mit einer 5 %igen wässrigen $NaHCO_3$-Lösung gewaschen, mit $MgSO_4$ getrocknet und durch Kieselgel filtriert. Die Chromatographie des Rohproduktes an 20 g Kieselgel (Ethylacetat/Hexan 1:4 [100 ml] und 2:3 [250 ml]) ergab 98 mg (49% d.Th.) 5-O-tert.Butyldimethylsilyl-13β-brom-milbemycin D und 50 mg (30% d.Th.) 13β-Brom-milbemycin D.

Die Behandlung des t-Butyldimethylsilyläther-Derivats mit 2 ml einer 1%igen Lösung von p-Toluolsulfonsäure in Methanol (1 Std. bei Raumtemperatur) gefolgt von der Aufarbeitung in Diethylether mit 5%iger wässriger $NaHCO_3$-Lösung und der Chromatographie an Kieselgel (Ethylacetat/Hexan 2:3) ergab in quantitativer Ausbeute 13β-Brom-Milbemycin D.

$^1$H-NMR (300 MHz; $CDCl_3$; TMS):
1,68 (s)($C_{14}CH_3$);
1,87 (s)($C_4CH_3$);
4,30 (d; J=10,7)($C_{13}H$).

Massenspektrum m/e: 636, 634 ($M^+$; $C_{33}H_{47}BrO_7$), 508, 506, 427, 209, 149, 41. Auf analoge Weise erhält man 13β-Brom-milbemycin $A_4$ mit Massenspektrum m/e: 622, 620.

29. Herstellung von 5-O-tert.Butyldimethylsilyl-13β-jod-milbemycin D und von 13β-Jod-milbemycin D

Eine Lösung von 526 mg (0,776 mmol) 5-O-tert.Butyldimethylsilyl-15-hydroxi-$\Delta^{13,14}$-milbemycin D,493 mg (1,884 mmol) Triphenylphosphin, 128 mg (1,884 mmol) Imidazol und 292 mg (1,149 mmol) Jod in 10 ml trockenem Dichlormethan wurde 20 min. bei Raumtemperatur unter Argon gerührt. Das Gemisch wurde mit Diethylether verdünnt, mit 5%iger

wässriger $NaHCO_3$-Lösung gewaschen, und die organische Phase wurde mit $MgSO_4$ getrocknet. Die Chromatographie des Rohproduktes an 75 g Kieselgel (Ethylacetat/Hexan 1:12 [500 ml]) ergab 390 mg (64%) 5-O-tert.Butyldimethylsilyl-13β-jod-milbemycin D.

159 mg (0,20 mmol) dieser silylierten Verbindung wurden mit 2 ml einer 1%igen Lösung von p-Toluolsulfonsäure in Methanol 1 Std. lang bei Raumtemperatur gerührt und dann mit 5%iger wässriger $NaHCO_3$-Lösung und Diethylether aufgearbeitet. Die Säulenchromatographie an 20 g Kieselgel (Laufmittel Ethylacetat/Hexan 2:3) ergab 103 mg (76% d.Th.) 13β-Jod-milbemycin D.

$^1$H-NMR (300 MHz; $CDCl_3$; TMS):
1,64 (s)($C_{14}CH_3$);
1,82 (s)($C_4CH_3$)
4,52 (d; J=11,0)($C_{13}H$).

30. Herstellung von 5-O-tert.Butyldimethylsilyl-13β-chloro-milbemycin $A_4$ und von 13β-Chloro-milbemycin $A_4$.

Zu einer Lösung von 198 mg (0,295 mmol) 5-O-tert.Butyldimethylsilyl-15-hydroxi-$\Delta^{13,14}$-Milbemycin $A_4$ und 0,082 ml (=60 mg; 0,589 mmol) Triethylamin in 5 ml trockenem Dichlormethan wurden unter Argon bei -10°C unter Rühren 0,026 ml (=42 mg; 0,354 mmol) Thionylchlorid getropft. Nach 5 min. wurden 0,1 ml Methanol zugegeben. Das Gemisch wurde mit 5%iger wässriger $NaHCO_3$-Lösung und Diethylether (=Ether) aufgearbeitet. Die Chromatographie an 20 g Kieselgel (Ethylacetat/Hexan 1:19) ergab 96 mg (47%) 5-O-tert.Butyldimethylsilyl-13β-chloro-milbemycin $A_4$.

Dieses Produkt wurde mit 2 ml einer 1%igen Lösung von p-Toluolsulfonsäure in Methanol 1 Std. lang bei Raumtemperatur gerührt, mit 5%iger wässriger $NaHCO_3$-Lösung behandelt und an 20 g Kieselgel (Ethylacetat/Hexan 2:3) chromatographiert. Man erhielt 66 mg (82% d.Th.) 13β-Chlor-Milbemycin $A_4$.

$^1$H-NMR (250 MHz; $CDCl_3$; TMS):
1,67 (s)($C_{14}CH_3$)
3,10 (t)(J=ca. 7Hz)($C_{25}H$)
5,24 (d)(J=ca. 11Hz)($C_{13}H$)
Massenspektrum m/e: 576 ($M^+$, $C_{32}H_{45}O_7Cl$)
448, 442, 348, 279, 195, 167, 151.

31. Herstellung von 5-O-Methyldiphenylsilyl-13β-chloro-milbemycin-$A_3$ und von 13β-Chloromilbemycin $A_3$.

Nach der Vorschrift des Beispiels 30 werden aus 194 mg 5-O-Methyldiphenylsilyl-15-hydroxi-$\Delta^{13,14}$-Milbemycin $A_3$ und 0,025 ml $SOCl_2$ 122 mg 5-O-Methyldiphenylsilyl-13β-chloro-milbemycin $A_3$ und nach Abspaltung des Silylrests 83 mg 13β-Chloro-milbemycin $A_3$ erhalten.

$^1$H-NMR (250 MHz; $CDCl_3$; TMS):
1,64 (s) ($C_{14}CH_3$)
1,87 (s) ($C_4CH_3$)
4,08 (d) (J=11Hz) ($C_{13}H$).

32. Herstellung von 5-O-tert.Butyldimethylsilyl-13α-chloro-22,23-dihydroavermectin-B1a-aglykon und von 13α- chloro-22,23-dihydroavermectin-B1a-aglykon

Zu einer Lösung von 327 mg (0,467 mmol) 5-O-tert.Butyldimethylsilyl-22,23-dihydroavermectin-B1a-aglykon und 0,130 ml (=95 mg; 0,934 mmol) Triethylamin in 10 ml trockenem Dichlormethan wurden unter Argon bei -10°C 0,041 ml (=67 mg; 0,561 mmol) $SOCl_2$ unter Rühren getropft. Nach 5 min. wurden 0,1 ml Methanol zugegeben. Das Gemisch wurde mit 5%iger wässriger Na-Hydrogencarbonat-Lösung und Ether aufgearbeitet. Die Chromatographie des Rohprodukts an 20 g Kieselgel (Ethylacetat/Hexan 1:9) ergab 117 mg (50% d.Th.) 5-O-tert.Butyldimethylsilyl-13α-chloro-22,23-dihydroavermectin-B1a-aglykon .

Dieses Produkt wurde mit 2 ml einer 1%igen Lösung von p-Toluolsulfonsäure in $CH_3OH$ während 1 Std. Rühren bei Raumtemperatur entsilyiert, dann mit 5%iger wässriger Na-Hydrogencarbonat-Lösung und Ether aufgearbeitet und an 20 g Kieselgel chromatographiert (Ethylacetat/Hexan 2:3). Man erhielt 68 mg (69% d.Th.) 13α-Chloro-22,23-dihydro-avermectin-Bla-aglykon.

$^1$H-NMR (300 MHz; $CDCl_3$; TMS):
1,63 (s) ($C_{14}CH_3$)
1,87 (s) ($C_4CH_3$)
4,39 (s) ($W_{1/2}$=6Hz)($C_{13}H$).
Massenspektrum m/e: 604 ($M^+$, $C_{34}H_{49}O_7Cl$),
476, 348, 223, 195, 151.

33a. Herstellung von 5-O-tetr.Butyldimethylsilyl-13β-hydroxi-milbemycin D und von 13β-Hydroxi-milbemycin D

Eine Lösung besteht aus 286 mg (0,41 mmol) 5-O-tert.Butyldimethylsilyl-15-hydroxi-$\Delta^{13,14}$-milbemycin D und 209 mg (0,56 mmol) Pyridiniumdichromat (PDC) in 3 ml Dimethylformamid (DMF) wurde 30 min. bei Raumtemperatur gerührt. Anschliessend wurde 1 ml Isopropanol zugegeben, 5 min. weitergerührt und dann mit 50 ml Ether verdünnt. Nach weiteren 10 min. wurde das Gemisch durch Kieselgel filtriert und eingeengt. Bei der Chromatographie des Rohproduktes an 20 g Kieselgel (Ether/Hexan 1:2) wurden 165 mg (57%) 5-O-t-Butyldimethylsilyl-13β-hydroxi-Milbemycin D erhalten.

$^1$H-NMR (300 MHz; $CDCl_3$; TMS):
1,59 (br.s)($C_{14}CH_3$)
3,70 (d; J= 10)($C_{13}H$).

105 mg (0.153 mmol) der so gewonnenen Verbindung wurden mit 1 ml einer 1 %igen Lösung von p-Toluolsulfonsäure in Methanol 1 Std. bei Raumtemperatur gerührt. Das Gemisch wurde mit 20 ml Ether verdünnt, durch Kieselgel filtriert, eingeengt, und der Rückstand wurde an ca. 10 g Kieselgel chromatographiert (Aceton/Dichlormethan 1:4), wobei 73 mg (83%) 13β-Hydroxi-Milbemycin D erhalten wurden.

$^1$H-NMR (300 MHz; $CDCl_3$; TMS):
1,58 (br.s)$C_{14}CH_3$)
3,71 (d; J = 10)$C_{13}H$).

33b. Herstellung von 5-O-tert.-Butylmethylsilyl-13β-hydroxi-milbemycin $A_4$ und von 13β-Hydroxi-milbemycin $A_4$

Analog zu Vorschrift 33a erhält man 5-O-tert.-Butyldimethylsilyl-13β-hydroxi-milbemyxin (Massenspektrum m/e: 672), das nach Abspaltung der tert.-Butyldimethylsilylgruppe gemäss Vorschrift 33a zu 13β-Hydroxi-milbemycin $A_4$ führt (Massenspektrum m/e: 558).

34. Herstellung von 5-O-tert.Butyldimethylsilyl-13α-hydroxy-milbemycin D und von 13α-Hydroxi-milbemycin D.

Die selektive 13-Hydrierung des gemäss Beispiel 21 erhaltenen 5-O-tert.Butyldimethylsilyl-13-oxo-Milbemycin D mit $NaBH_4$ ergibt neben dem 5-O-tert.Butyldimethylsilyl-13β-hydroxy-Milbemycin D das 5-O-tert.Butyldimethylsilyl-13α-hydroxi-milbemycin D, aus dem durch Entsilylierung gemäss einem der vorstehenden Beispiele 13α-Hydroxi-milbemycin D erhalten wird. Entsprechend lassen sich 13α-Hydroxi-milbemycin $A_3$ und 13α-Hydroxi-milbemycin-$A_4$ herstellen; daneben entstehen ca. 5 % des 13β-Hydroxi-milbemycin $A_4$.

35. Herstellung von 5-O-tert.Butyldimethylsilyl-13α-fluor-milbemycin und von 13α-Fluor-milbemycin ($R_2$=$CH_3$, $C_2H_5$ oder iso$C_3H_7$).

Nach der Vorschrift des Beispiels 24 erhält man aus 5-O-tert.Butyldimethylsilyl-13α-hydroxi-milbemycin durch Reaktion mit DAST das 5-O-tert.Butyldimethylsilyl-13α-fluor-milbemycin und daraus durch milde Abspaltung des Silylrestes das 13α-Fluor-milbemycin.

36. Herstellung von 5-O-tert.Butyldimethylsilyl-13α-chlor-milbemycin und von 13α-Chlormilbemycin ($R_2$ = $CH_3$, $C_2H_5$, iso$C_3H_7$).

Nach der Vorschrift des Beispiels 27 erhält man aus 5-O-tert.Butyldimethylsilyl-13α-hydroxi-milbemycin durch Reaktion mit Thionylchlorid das 5-O-tert.Butyldimethylsilyl-13α-chlor-milbemycin und nach Abspaltung des Silylrestes 13α-Chlor-milbemycin, unter denen das 13α-Chlor-milbemycin $A_4$ ($R_2$ = Ethyl) wegen seiner hohen Wirksamkeit auf Endoparasiten besonders zu nennen ist.

37. Herstellung von 5-O-tert.Butyldimethylsilyl-13α-fluor-[bzw. 13β-fluor]-22,23-dihydro-avermectin-aglykon ($R_2$ = sek.$C_4H_9$) und von 13α-Fluor-22,23-dihydro-avermectin-aglykon sowie von 13β-Fluor-22,23-dihydro-avermectin-aglykon.

Eine Lösung von 0,044 ml (58 mg, 0,363 mmol) DAST in 0,4 ml trockenem Dichlormethan wurde bei -78° unter Rühren zu einer Lösung von 127 mg (0,181 mmol) 5-O-tert.Butyldimethylsilyl-13-desoxi-22,23-dihydro-avermectin-aglykon in 2 ml trockenem Dichlormethan getropft. Nach 15 min. wurde eine Lösung von 0,5 ml Ethanol in 3 ml Ether zugegeben, mit Ether verdünnt und die organische Phase mit 5% Natriumhydrogencarbonatlösung gewaschen, mit $MgSO_4$ getrocknet und eingeengt. Die Chromatographie des Rohproduktes an 20 g Kieselgel (Ethylacetat/Hexan 1:9) ergab 24 mg (19%) 5-O-tert.Butyldimethylsilyl-13β-fluor-22,23-dihydroavermectin-aglykon und 55 mg (43%) 5-O-tert.Butyldimethylsilyl-13α-fluor-22,23-dihydro-avermectin-aglykon.

55 mg der 13α-Fluoro-Verbindung wurden mit 1 ml einer 1%igen Lösung von p-Toluolsulfonsäure in Methanol 1 Std. bei Raumtemperatur gerührt, mit 20 ml Ether verdünnt und durch Kieselgel filtriert. Die Chromatographie an 10 g Kieselgel (Ethylacetat/Hexan 2:3) ergab 33 mg (74%) 13α-Fluor-22,23-dihydro-avermectin-aglykon, die als Nematozid besonders wichtig ist.

$^1$H-NMR (300 MHz; $CDCl_3$; TMS):
4,40 (dxd; $J_1$ = 47,7 Hz; $J_2$ = 10,0 Hz)($C_{13}H$)
1,61 (m, $W_{1/2}$ = 4,5 Hz)($C_{14}CH_3$).

Bei der Behandlung von 24 mg der 13β-Fluoro-Verbindung mit 1 ml 1%iger p-Toluolsulfonsäure in Methanol wurden 6 mg 13β-Fluoro-22,23-dihydro-avermectin-aglykon gewonnen.

$^1$H-NMR (300 MHz; $CDCl_3$; TMS):
4,71 (d; J = 47,6 Hz; $W_{1/2}$ = 6,5 Hz)($C_{13}H$)
1,49 (m; $W_{1/2}$= 4 Hz,)($C_{14}CH_3$).

38. Herstellung von 5-tert.-Butyldimethylsilyl-13α-hydroxy-Milbemycin D.

Zu einer Lösung von 8 mg (0,0117 mmol) 5-t-Butyldimethylsilyl-13-oxo-Milbemycin D in 1 ml Ethanol wurde eine Suspension von Natriumborhydrid (1 mg, 0,0264 mmol) in Ethanol (0,1 ml) unter Rühren zugetropft. Nach 10 Min. wurde $NH_4Cl$-Lösung (ges. wässrig, 0,05 ml) zugegeben, mit Ether verdünnt, mit $MgSO_4$ getrocknet, und durch Kieselgel filtriert. Die Chromatographie des Rohproduktes an 20 g Kieselgel (Essigsäureethylester/Hexan 1:4 [100 ml]) ergab 4 mg (50 %) 5-t-Butyl-dimethylsilyl-13α-hydroxy-Milbemycin D.

$^1H$-NMR (300 MHz, $CDCl_3$): 1,53 ppm (s, $CH_3$-C(14)); 1,79 ppm (2, $CH_3$-C(4)); 4,00 ppm (s, $w_{1/2}$ = 5 Hz, H-C(13)).

Gemäss den Beispielen 22 ff. gegebenen Vorschriften lassen sich folgenden Verbindungen der Formel I herstellen, wobei die 5-OH-Position durch Silylierung, Acylierung, Benzylierung oder anderweitig geschützt sein kann.

[Sil = $-Si(CH_3)_2-C(CH_3)_3$ oder $-Si(CH_3)(C_6H_5)_2$] .

| Verb.Nr. | $R_1$ | $R_2$ | R | Herst. Beispiel Nr. |
|---|---|---|---|---|
| 1.1 | Sil | iso$C_3H_7$ | β-F | 24 |
| 1.2 | Sil | iso$C_3H_7$ | β-Cl | 27 |
| 1.3 | Sil | iso$C_3H_7$ | β-Br | 28 |
| 1.4 | Sil | iso$C_3H_7$ | β-J | 29 |
| 1.5 | Sil | iso$C_3H_7$ | β-OH | 33a (34) |
| 1.6 | H | iso$C_3H_7$ | β-F | 24 |
| 1.7 | H | iso$C_3H_7$ | β-Cl | 27 |
| 1.8 | H | iso$C_3H_7$ | β-Br | 28 |
| 1.9 | H | iso$C_3H_7$ | β-J | 29 |
| 1.10 | H | iso$C_3H_7$ | β-OH | 33a (34) |
| 1.11 | Sil | iso$C_3H_7$ | α-F | 35 |
| 1.12 | Sil | iso$C_3H_7$ | α-Cl | 36 |
| 1.13 | Sil | iso$C_3H_7$ | α-Br | |
| 1.14 | Sil | iso$C_3H_7$ | α-J | |
| 1.15 | Sil | iso$C_3H_7$ | α-OH | 34 |
| 1.16 | H | iso$C_3H_7$ | α-F | 35 |
| 1.17 | H | iso$C_3H_7$ | α-Cl | 36 |
| 1.18 | H | iso$C_3H_7$ | α-Br | |
| 1.19 | H | iso$C_3H_7$ | α-J | |
| 1.20 | H | iso$C_3H_7$ | α-OH | 34 |
| 1.21 | Acetyl | iso$C_3H_7$ | β-Cl | |
| 1.22 | Benzyl | iso$C_3H_7$ | β-Cl | |
| 2.1 | Sil | $C_2H_5$ | β-F | 25 |
| 2.2 | Sil | $C_2H_5$ | β-Cl | 30 |
| 2.3 | Sil | $C_2H_5$ | β-Br | 28b |
| 2.4 | Sil | $C_2H_5$ | β-J | |
| 2.5 | Sil | $C_2H_5$ | β-OH | (34) |

| Verb.Nr. | $R_1$ | $R_2$ | R | Herst.-Beispiel Nr. |
|---|---|---|---|---|
| 2.6 | H | $C_2H_5$ | β-F | 25 |
| 2.7 | H | $C_2H_5$ | β-Cl | 30 |
| 2.8 | H | $C_2H_5$ | β-Br | 28b |
| 2.9 | H | $C_2H_5$ | β-J | |
| 2.10 | H | $C_2H_5$ | β-OH | 33b |
| 2.11 | Sil | $C_2H_5$ | α-F | 35 |
| 2.12 | Sil | $C_2H_5$ | α-Cl | 36 |
| 2.13 | Sil | $C_2H_5$ | α-Br | |
| 2.14 | Sil | $C_2H_5$ | α-J | |
| 2.15 | Sil | $C_2H_5$ | α-OH | (34) |
| 2.16 | H | $C_2H_5$ | α-F | 35 |
| 2.17 | H | $C_2H_5$ | α-Cl | 36 |
| 2.18 | H | $C_2H_5$ | α-Br | |
| 2.19 | H | $C_2H_5$ | α-J | |
| 2.20 | H | $C_2H_5$ | α-OH | (34) |
| 2.21 | Acetyl | $C_2H_5$ | β-Cl | |
| 2.22 | Benzyl | $C_2H_5$ | β-Cl | |

Entsprechend lassen sich, wie in den Beispielen 24 ff und in der weiter oben gegebenen Beschreibung erläutert , die 5-OH-geschützen Milbemycin-Derivate der Formel I der Reihen $A_3$ ($R_2$ = $CH_3$) und "22,23-Dihydro-avermectin" ($R_2$ = sek.$C_4H_9$) gewinnen. Aus diesen sind nach Abspaltung der Schutzgruppe die entsprechenden 5-Hydroxi-milbemycine darstellbar ($R_1$ = H).

| Verb.Nr. | $R_2$ | R | Herst.-Beispiel Nr. |
|---|---|---|---|
| 3.1 | $CH_3$ | β-F | 26 |
| 3.2 | $CH_3$ | β-Cl | 31 |
| 3.3 | $CH_3$ | α-F | 35 |
| 3.4 | $CH_3$ | α-Cl | 36 |
| 3.5 | $CH_3$ | β-OH | |
| 3.6 | $CH_3$ | β-OH | (34) |

| Verb.Nr. | $R_2$ | R | Herst.-Beispiel Nr. |
|---|---|---|---|
| 4.1 | sek.$C_4H_9$ | β-F | 37 |
| 4.2 | sek.$C_4H_9$ | α-F | 37 |
| 4.3 | sek.$C_4H_9$ | α-Cl | 32 |
| 4.4 | sek.$C_4H_9$ | β-OH | |

Nach der Vorschrift des Beispiels 23 lassen sich die wichtigen Zwischenprodukte der Formel IV gewinnen, worin $R_1$ Wasserstoff bedeutet.

| Verb.Nr. | $R_2$ |
|---|---|
| Z.1 | $CH_3$ |
| Z.2 | $C_2H_5$ |
| Z.3 | iso$C_3H_7$ |
| Z.4 | sek.$C_4H_9$ |

Formulierungsbeispiele für Wirkstoffe der Formel I

(% = Gewichtsprozent)

| Spritzpulver | a) | b) | c) |
|---|---|---|---|
| Wirkstoff | 25% | 50% | 75% |
| Na-Ligninsulfonat | 5% | 5% | - |
| Na-Laurylsulfat | 3% | - | 5% |
| Na-Diisobutylnapthalinsulfonat | - | 6% | 10% |
| Octylphenolpolyäthylenglykoläther (7-8 Mol AeO) | - | 2% | - |
| Hochdisperse Kiselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Emulsions-Konzentrat

| | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

Falls eine Verbindung der Formel I bzw. entsprechende Mittel zur Bekämpfung von endoparasitären Nematoden bei Haus- und Nutztieren, wie Rindern, Schafen, Ziegen, Katzen und

Hunden, verwendet werden, können sie den Tieren sowohl als Einzeldosis wie auch wiederholt verabreicht werden, wobei die einzelnen Gaben je nach Tierart vorzugsweise zwischen 0.01und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew.-%. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, kann eine Verbindung der Formel I bzw. sie enthaltende Mittel an Tieren auch subcutan injiziert oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

### B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 12,5 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24° C und 60 % relativer Luftfeuchtigkeit durchgeführt. Auswertungen und Zwischenauswertungen auf moribunde Tiere, Wachstum der Larven und Frassschäden erfolgen nach 24 Std., 48 Std.

und 72 Stunden. Verbindungen der Formel I, so z.B. die Verbindungen Nr. 1.6, 4.1, 4.2 und 4.3, erzielen bereits nach 24 Std. eine vollständige Abtötung.

B-2. Wirkung gegen pflanzenschädigende Akariden

OP-sensible Tetranychus urticae

Die Primärblätter von Bohnenpflanzen (Phaseolus vulgaris) werden 16 Std. vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die 1,6 ppm der zu prüfenden Verbindung enthält.
Die Temperatur in der Gewächshauskabine beträgt ca. 25° C.

Nach sieben Tagen wird unter dem Binokular auf Prozentsatz mobiler Stadien (Adulte und Nymphen) und auf vorhandene Eier ausgewertet.

Verbindungen der Formel I erzielen bei dieser Konzentration eine mindestens 98%ige Abtötung.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca, 50°C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Verbindungen der Formel I, wie z.B. Nr. 1.6, 1.7, 1.8, 1.9, 1.10, 2.7, 2.20, 3.2, 4.1, 4.2 und 4.3 erzielen bei 250 ppm eine vollständige, bei 125 ppm eine mindestens 90%ige Abtötung.

B-4. Akarizide Wirkung gegen Boophilus microplus (Biarra-Stamm)

Auf einer PVC-Platte wird waagerecht ein Klebestreifen so befestigt, dass darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel

1 µl einer Flüssigkeit injiiziert, die eine 1:1-Mischung von Polyethylenglykol und Aceton darstellt und in der eine bestimmte Wirkstoffmenge von wahlweise 1, 0.1 oder 0.01 µg pro Zecke gelöst ist. Kontrolltiere erhalten eine wirkstoffreie Injektion. Nach der Behandlung werden die Tiere unter Normalbedingungen in einem Insektarium bei ca. 28° C und 80 % rel. Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der nach 30 Tagen 9 von 10 Zeckenweibchen (= 90 %) nicht schlupffähige Eier ablegen.
Die Verbindungen der Formel I erreichen bei 1 µg sämtlichst eine volle Wirkung (keine schlupffähigen Eier).
Die Verbindungen Nr. 1.6, 1.7, 1.8, 4.1 und 4.2 erzielen eine $IR_{90}$ von 0.1 µg.

## B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trichostrongylus colubriformis künstlich infiziert worden ist. Pro Dosis werden 3 Tiere verwendet.
Jedes Schaf wird nur einmal mit einer einzigen Dosis von 0,2 mg A.S. /kg Körpergewicht behandelt. Die Evaluierung nach 7 Tagen erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen Nr. 1.6 , 1.7, 1.8, 1.9, 1.16, 1.17, 2.6, 2.7, 2.16, 2.17, 3.1-3.4, 4.2 behandelt werden, zeigen im Vergleich zu unbehandelten, aber infizierten Vergleichsgruppen keinen Nematodenbefall (= komplette Reduktion der Wurmeier im Kot).

Patentansprüche:

1. 13-substituierte Milbemycin-Derivate der Formel I*

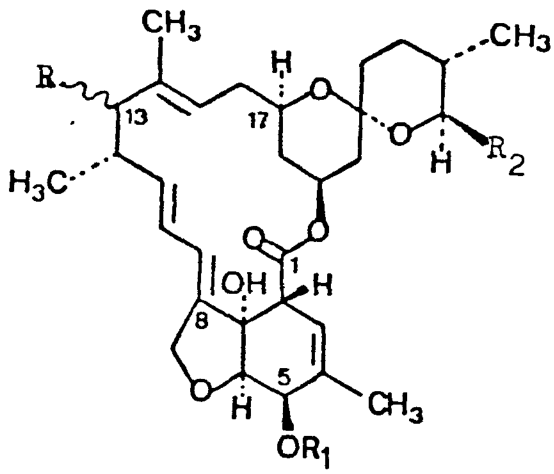

I*

worin

$R_1$ Wasserstoff, eine Silyl- oder Acylgruppe bedeutet, und

a) R α-Hydroxi, Fluor, Chlor oder Jod ist, wenn $R_2$ Methyl oder Ethyl bedeutet,
oder

b) R β-Hydroxi, α-Chlor, Fluor, Brom oder Jod ist, wenn $R_2$ Isopropyl oder sek.Butyl bedeutet,
oder

c) R β-Chlor ist, wenn $R_2$ Isopropyl bedeutet.

2. Verbindungen der Formel I* gemäss Anspruch 1, bei denen $R_1$ Wasserstoff darstellt, und

a') R α-Fluor, β-Fluor, α-Chlor, β-Chlor, α-Jod oder β-Jod ist, wenn $R_2$ Ethyl bedeutet, oder

b') R β-Hydroxi, α-Chlor, β-Chlor, α-Fluor, β-Fluor, α-Brom, β-Brom, α-Jod oder β-Jod ist, wenn $R_2$ Isopropyl bedeutet.

3. 13β-Chlor-Milbemycin D gemäss Anspruch 1.

4. 13β-Chlor-Milbemycin $A_4$ gemäss Anspruch 1.

5. 13β-Fluor-Milbemycin D gemäss Anspruch 1.

6. 13β-Fluor-Milbemycin $A_4$ gemäss Anspruch 1.

7. 13β-Hydroxi-Milbemycin D gemäss Anspruch 1.

8. 13β-Hydroxi-Milbemycin $A_4$ gemäss Anspruch 1.

9. 13α-Fluor-22,23-dihydro-avermectin-aglykon gemäss Anspruch 1.

10. 13α-Fluor-Milbemycin D gemäss Anspruch 1.

11. Verfahren zur Herstellung einer Verbindung der Formel I

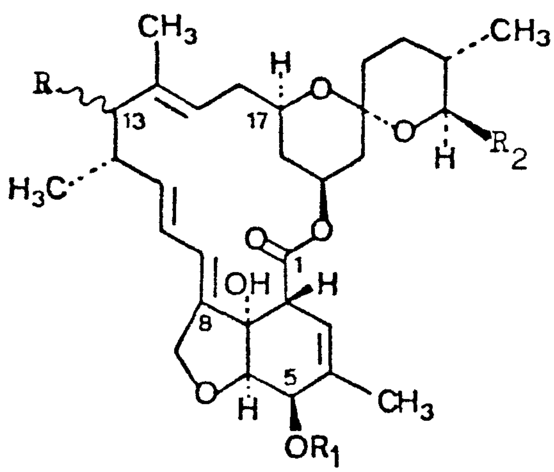

I

worin R in der 13β-Position steht und Fluor, Chlor, Brom oder Jod darstellt,

$R_1$ Wasserstoff, eine Silyl- oder Acylgruppe ist, und

$R_2$ Methyl, Ethyl, Isopropyl oder sek.Butyl bedeutet, gekennzeichnet durch 13-β-Halogenierung einer Verbindung der Formel II

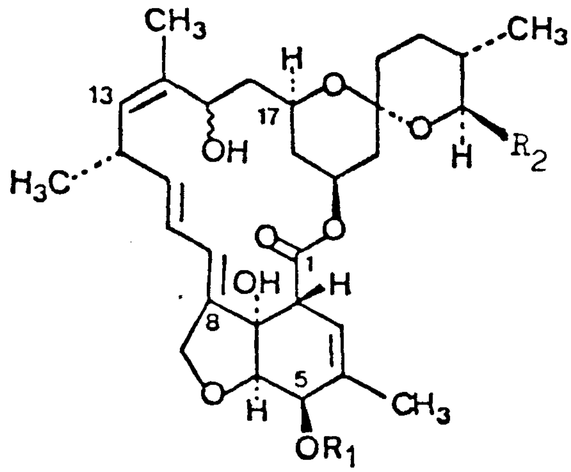

II

worin $R_2$ die für Formel I genannte Bedeutung hat, und $R_1$ eine 5-OH-Schutzgruppe darstellt, mit zur Einführung von Halogen in die 13β-Position geeigneten Halogenierungsmitteln, und, sofern gewünscht, anschliessende Entfernung der Schutzgruppe aus der 5-Position.

12. Verfahren zur Herstellung von Verbindungen der Formeln I und IV

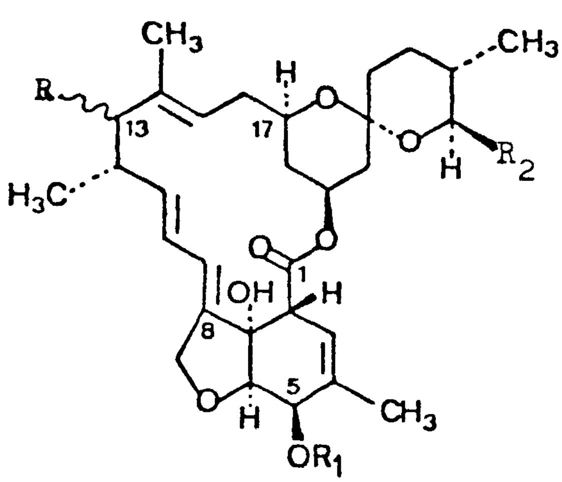

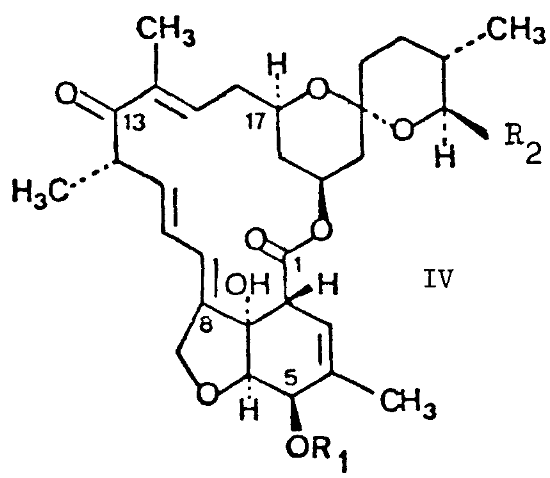

IV

worin R eine β-Hydroxi-Gruppe bedeutet, $R_1$ Wasserstoff, eine Silyl- oder Acylgruppe ist, und $R_2$ Methyl, Ethyl, Isopropyl oder sek.Butyl bedeutet, gekennzeichnet durch Reaktion einer Verbindung der Formel II

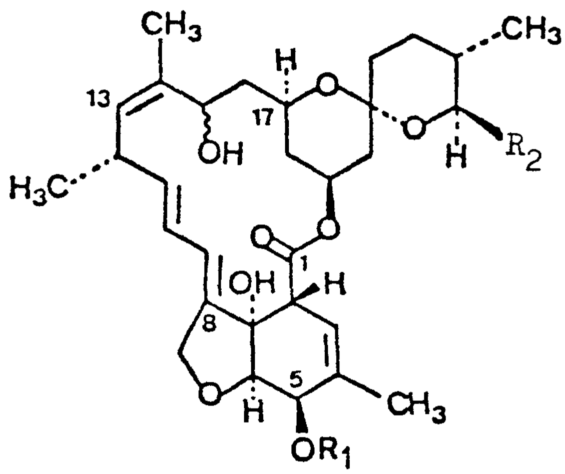

II

worin $R_2$ die für Formel I genannte Bedeutung hat, und $R_1$ eine 5-OH-Schutzgruppe darstellt, mit Chromat-, Halochromat- oder Dichromat-Ionen bei -10°C bis +60°C, bevorzugt +10°C bis +40°C, und Abtrennung der 13 Oxo-Verbindungen der Formel IV.

13. Verfahren zur Herstellung einer Verbindung der Formel I

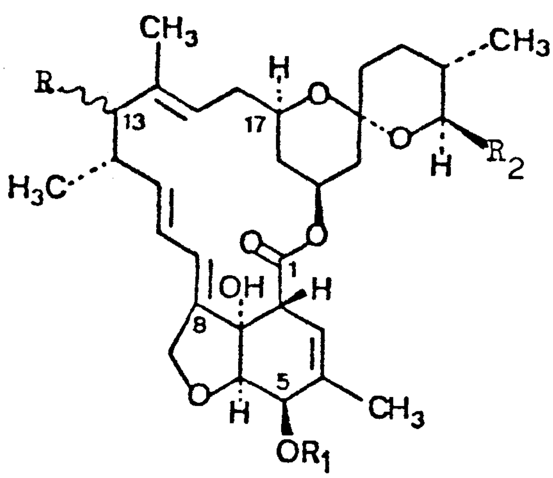

I

worin R eine α-Hydroxi-Gruppe bedeutet, $R_1$ Wasserstoff, eine Silyl- oder Acylgruppe ist, und $R_2$ Methyl, Ethyl, Isopropyl oder sek.Butyl bedeutet, gekennzeichnet durch selektive Hydrierung einer Verbindung der Formel IV in 13-Position

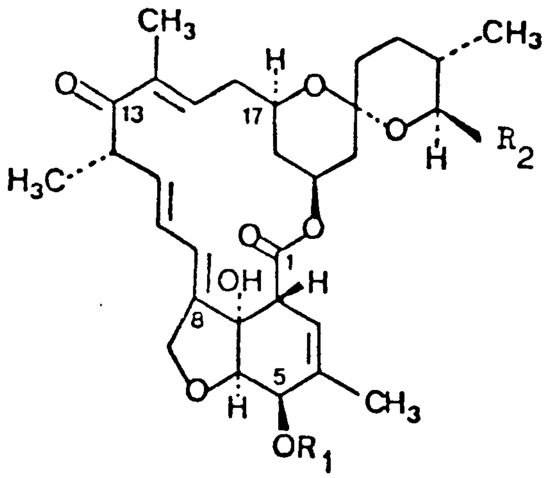

IV

worin $R_2$ die für Formel I genannte Bedeutung hat, und $R_1$ eine 5-OH-Schutzgruppe darstellt, mit einem Metallhydrid und, sofern gewünscht, Abtrennung der 13α-Hydroxi-Isomeren von den 13β-Hydroxi-Isomeren.

14. Verfahren zur gleichzeitigen Herstellung von 13α-Fluor- und 13β-Fluor-milbemycin-Derivaten der Formel I

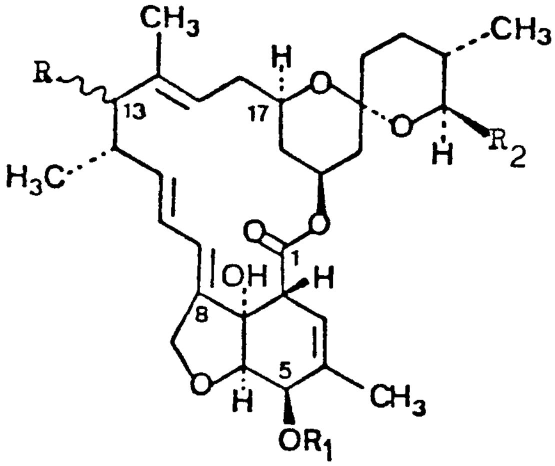

I

worin R Fluor darstellt, $R_1$ Wasserstoff, eine Silyl- oder Acylgruppe ist, und $R_2$ Methyl, Ethyl, Isopropyl oder sek.Butyl bedeutet, gekennzeichnet durch Reaktion einer Verbindung der Formel I, worin R die 13α-Hydroxi-Gruppe

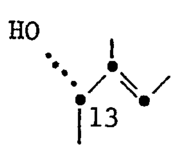

bedeutet, mit Schwefeltetrafluorid, $SF_4$, oder Selentetrafluorid, $SeF_4$, oder einem ihrer reaktionsanalogen Derivate, bevorzugt Diethylaminoschwefeltrifluorid (DAST).

15. Verfahren zur Herstellung von 13α-Chlor-13α-Brom- und 13α-Jod-milbemycin-Derivaten der Formel I

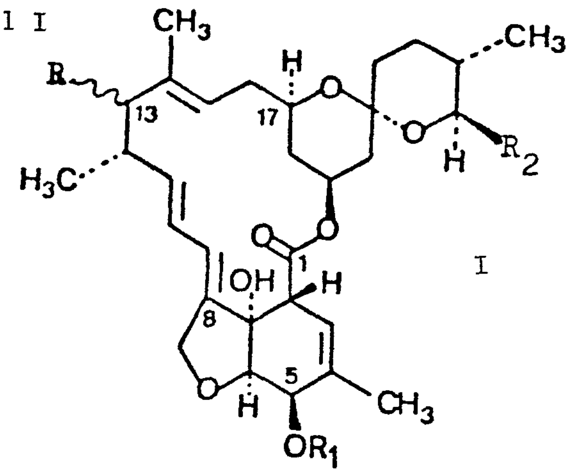

I

worin R α-Cl, α-Br oder α-J bedeutet, $R_1$ Wasserstoff, eine Silyl- oder Acylgruppe ist, und $R_2$ Methyl, Ethyl, Isopropyl oder sek.Butyl bedeutet, gekennzeichnet durch Reaktion einer Verbindung der Formel I, worin R die 13α-Hydroxi-Gruppe bedeutet, mit wahlweise Thionylchlorid bzw. Phosphortribromid bzw. Jod/Triphenylphosphin/Imidazol.

16. Schädlingsbekämpfungsmittel gegen Ekto- und Endoparasiten, das neben üblichen Trägerstoffen und/oder Verteilungsmitteln als mindestens einen Wirkstoff eine Verbindung der Formel I

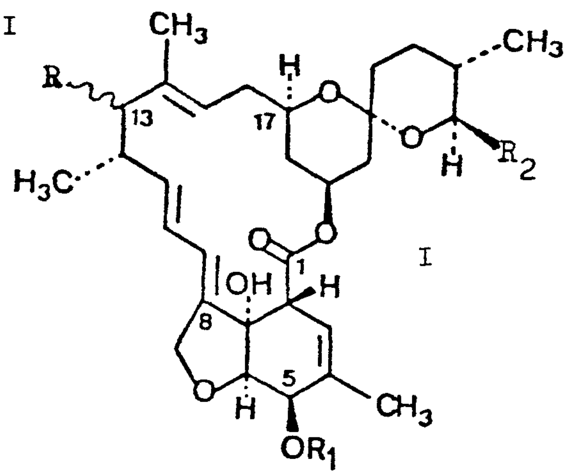

I

enthält, worin

$R_1$ Wasserstoff, eine Silyl- oder Acylgruppe ist,

$R_2$ Methyl, Ethyl, Isopropyl oder sek.Butyl bedeutet, und R Fluor, Chlor, Brom, Jod oder eine Hydroxigruppe darstellt.

17. Mittel gemäss Anspruch 16, das als Wirkstoff eine Verbindung der Formel I* gemäss Anspruch 1 enthält.

18. Mittel gemäss Anspruch 17, das als Wirkstoff eine Verbindung der Formel I* gemäss einem der Ansprüche 2 bis 10 enthält.

19. Verwendung einer Verbindung der Formel I gemäss einem der Ansprüche 16 oder 1 bis 10 zur Bekämpfung von Ekto- und Endoparasiten an Pflanzen und Tieren.

20. Verwendung gemäss Anspruch 19 zur Bekämpfung von Endoparasiten im Warmblüter.

21. Verwendung gemäss Anspruch 20, wobei die Endoparasiten Nematoden sind.

22. Verfahren zur Bekämpfung von Schädlingen an Tieren und Pflanzen, dadurch gekennzeichnet, dass man eine Verbindung der Formel I gemäss einem der Ansprüche 16 oder 1 bis 10 auf oder in die Tiere bzw. Pflanzen appliziert.

23. 13-Oxo-Milbemycin-Derivate der Formel IV

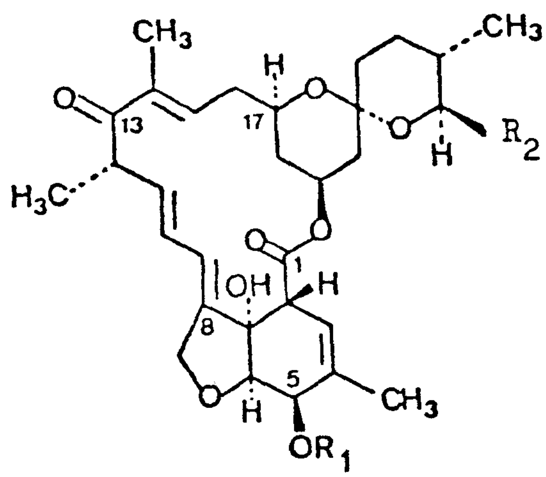

IV

worin $R_1$ Wasserstoff, eine Silyl- oder Acylgruppe und $R_2$ Methyl, Ethyl, Isopropyl oder sek.Butyl bedeuten.

24. Verfahren zur Herstellung von 13-Oxo-Verbindungen der Formel IV

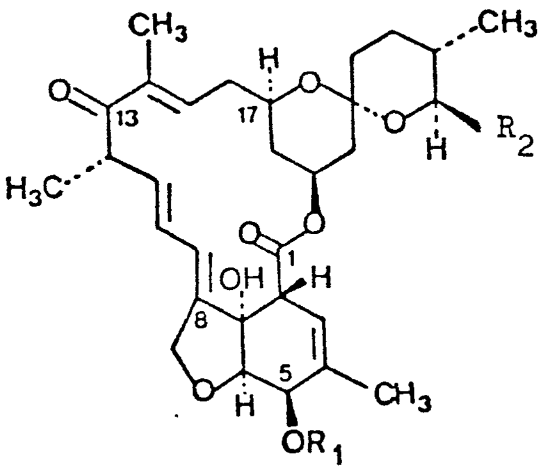

IV

worin

$R_1$ Wasserstoff, eine Silyl- oder Acylgruppe,

$R_2$ Methyl, Ethyl, Isopropyl oder sek.Butyl, und

R Fluor, Chlor, Brom, Jod oder eine Hydroxigruppe bedeutet, dadurch gekennzeichnet, dass man ein 13β-Hydroxi-Milbemycin der Formel I

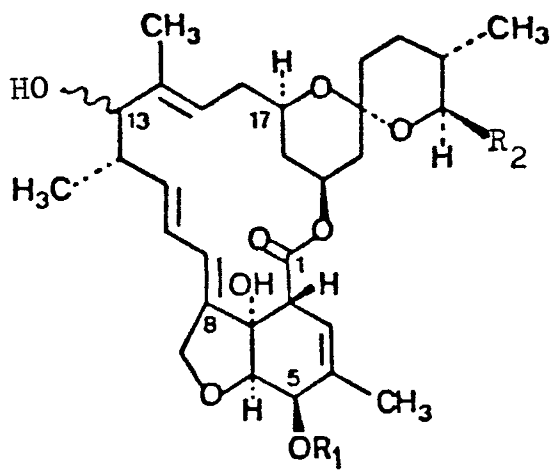

I

worin $R_1$ und $R_2$ wie oben definiert sind, bei Temperaturen von -100°C bis +60°C mit aktiviertem Dimethylsulfid oder Dimethylsulfoxid oxidiert.

FO 7.5/HL/gs*

0180539

Europäisches Patentamt

# EUROPÄISCHER RECHERCHENBERICHT

Nummer der Anmeldung

EP 85 81 0414

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-4 093 629 (M.H. FISHER)<br>* Spalte 6 *<br>--- | 1-10, 16-22 | C 07 D 493/22<br>A 01 N 43/22 //<br>(C 07 D 493/22<br>C 07 D 313:00<br>C 07 D 311:00<br>C 07 D 311:00<br>C 07 D 307:00 ) |
| X | FR-A-2 387 231 (MERCK & CO.)<br>* Seite 13, Zeilen 15-24 *<br>--- | 1-10, 16-22 | |
| X | EP-A-0 074 758 (MERCK & CO.)<br>* Seite 18, Zeilen 10-14 *<br>--- | 1-10, 16-22 | |
| X | EP-A-0 002 615 (MERCK)<br>* Seiten 4-18 *<br>--- | 1-10, 16-22 | RECHERCHIERTE SACHGEBIETE (Int. Cl.4) |
| Y | TETRAHEDRON LETTERS, Band 24, Nr. 48, 1983, Seiten 5333-5336, Pergamon Press Ltd., Oxford, GB; H. MROZIK et al.: "Synthesis of milbemycins from avermectins"<br>* Seiten 5333-5334 *<br>--- | 1-10, 16-22 | C 07 D 293/00<br>A 01 N 43/00<br>A 61 K 31/00 |
| Y | US-A-4 285 963 (B. ARISON)<br>* Spalte 2, Formel I; Spalte 10 *<br>----- | 1-10, 16-23 | |

Der vorliegende Recherchenbericht wurde für alle Patentansprüche erstellt.

| Recherchenort | Abschlußdatum der Recherche | Prüfer |
|---|---|---|
| DEN HAAG | 25-02-1986 | VERHULST W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument